# EUROPEAN PATENT APPLICATION

(11) **EP 3 933 052 A1**
(43) Date of publication of application: **05.01.2022**
(21) Application number: 20305759.1
(22) Date of filing: 03.07.2020
(51) Int. Cl.: C12Q 1/70

(54) **RAPID DETECTION KIT FOR HUMAN PATHOGENIC CORONAVIRUSES : BETACORONAVIRUS GROUP B/C AND SARS-COV-2**

(71) Applicant: Institut Pasteur, 75724 Paris Cédex 15 (FR)
(72) Inventor: VANHOMWEGEN, Jessica, 75724 PARIS Cedex 15 (FR); MANUGUERRA, Jean-Claude, 75724 PARIS Cedex 15 (FR)
(74) Representative: Plasseraud IP

(57) **Abstract**

The invention relates to reagents and methods for the rapid detection of the presence and/or absence of SARS-CoV-2 or other betacoronavirus group B/C nucleic acid in a sample and their use for the diagnosis of an infection or the detection of an environmental contamination by SARS-CoV-2 or other betacoronavirus group B/C.

SEQUENCE LISTING

<110>  INSTITUT PASTEUR

<120>  RAPID DETECTION KIT FOR HUMAN PATHOGENIC CORONAVIRUSES :
       BETACORONAVIRUS GROUP B/C AND SARS-CoV-2

<130>  BEP200632 EP

<160>  224

<170>  PatentIn version 3.5

<210>  1
<211>  1260
<212>  DNA
<213>  SARS-CoV-2

<400>  1
atgtctgata atggacccca aaatcagcga aatgcacccc gcattacgtt tggtggaccc      60

tcagattcaa ctggcagtaa ccagaatgga gaacgcagtg gggcgcgatc aaaacaacgt     120

cggccccaag gtttacccaa taatactgcg tcttggttca ccgctctcac tcaacatggc     180

aaggaagacc ttaaattccc tcgaggacaa ggcgttccaa ttaacaccaa tagcagtcca     240

gatgaccaaa ttggctacta ccgaagagct accagacgaa ttcgtggtgg tgacggtaaa     300

atgaaagatc tcagtccaag atggtatttc tactacctag gaactgggcc agaagctgga     360

cttccctatg gtgctaacaa agacggcatc atatgggttg caactgaggg agccttgaat     420

acaccaaaag atcacattgg cacccgcaat cctgctaaca atgctgcaat cgtgctacaa     480

cttcctcaag gaacaacatt gccaaaaggc ttctacgcag aagggagcag aggcggcagt     540

caagcctctt ctcgttcctc atcacgtagt cgcaacagtt caagaaattc aactccaggc     600

agcagtaggg gaacttctcc tgctagaatg gctggcaatg gcggtgatgc tgctcttgct     660

ttgctgctgc ttgacagatt gaaccagctt gagagcaaaa tgtctggtaa aggccaacaa     720

caacaaggcc aaactgtcac taagaaatct gctgctgagg cttctaagaa gcctcggcaa     780

aaacgtactg ccactaaagc atacaatgta acacaagctt tcggcagacg tggtccagaa     840

caaacccaag gaaattttgg ggaccaggaa ctaatcagac aaggaactga ttacaaacat     900

tggccgcaaa ttgcacaatt tgcccccagc gcttcagcgt tcttcggaat gtcgcgcatt     960

ggcatggaag tcacaccttc gggaacgtgg ttgacctaca caggtgccat caaattggat    1020

gacaaagatc caaatttcaa agatcaagtc attttgctga ataagcatat tgacgcatac    1080

aaaacattcc caccaacaga gcctaaaaag gacaaaaaga agaaggctga tgaaactcaa    1140

gccttaccgc agagacagaa gaaacagcaa actgtgactc ttcttcctgc tgcagatttg    1200

gatgatttct ccaaacaatt gcaacaatcc atgagcagtg ctgactcaac tcaggcctaa    1260

<210>  2

<211>  2795
<212>  DNA
<213>  SARS-CoV-2

<400>  2
tcagctgatg cacaatcgtt tttaaacggg tttgcggtgt aagtgcagcc cgtcttacac      60

cgtgcggcac aggcactagt actgatgtcg tatacagggc ttttgacatc tacaatgata     120

aagtagctgg ttttgctaaa ttcctaaaaa ctaattgttg tcgcttccaa gaaaaggacg     180

aagatgacaa tttaattgat tcttactttg tagttaagag acacactttc tctaactacc     240

aacatgaaga aacaatttat aatttactta aggattgtcc agctgttgct aaacatgact     300

tctttaagtt tagaatagac ggtgacatgg taccacatat atcacgtcaa cgtcttacta     360

aatacacaat ggcagacctc gtctatgctt taaggcattt tgatgaaggt aattgtgaca     420

cattaaaaga aatacttgtc acatacaatt gttgtgatga tgattatttc aataaaaagg     480

actggtatga ttttgtagaa aacccagata tattacgcgt atacgccaac ttaggtgaac     540

gtgtacgcca agctttgtta aaaacagtac aattctgtga tgccatgcga aatgctggta     600

ttgttggtgt actgacatta gataatcaag atctcaatgg taactggtat gatttcggtg     660

atttcataca aaccacgcca ggtagtggag ttcctgttgt agattcttat tattcattgt     720

taatgcctat attaaccttg accagggctt taactgcaga gtcacatgtt gacactgact     780

taacaaagcc ttacattaag tgggatttgt taaaatatga cttcacggaa gagaggttaa     840

aactctttga ccgttatttt aaatattggg atcagacata ccacccaaat tgtgttaact     900

gtttggatga cagatgcatt ctgcattgtg caaactttaa tgttttattc tctacagtgt     960

tcccacctac aagttttgga ccactagtga gaaaaatatt tgttgatggt gttccatttg    1020

tagtttcaac tggataccac ttcagagagc taggtgttgt acataatcag gatgtaaact    1080

tacatagctc tagacttagt tttaaggaat tacttgtgta tgctgctgac cctgctatgc    1140

acgctgcttc tggtaatcta ttactagata aacgcactac gtgcttttca gtagctgcac    1200

ttactaacaa tgttgctttt caaactgtca aacccggtaa ttttaacaaa gacttctatg    1260

actttgctgt gtctaagggt ttctttaagg aaggaagttc tgttgaatta aaacacttct    1320

tctttgctca ggatggtaat gctgctatca gcgattatga ctactatcgt tataatctac    1380

caacaatgtg tgatatcaga caactactat ttgtagttga agttgttgat aagtactttg    1440

attgttacga tggtggctgt attaatgcta accaagtcat cgtcaacaac ctagacaaat    1500

cagctggttt tccatttaat aaatggggta aggctagact ttattatgat tcaatgagtt    1560

atgaggatca agatgcactt ttcgcatata caaaacgtaa tgtcatccct actataactc    1620

aaatgaatct taagtatgcc attagtgcaa agaatagagc tcgcaccgta gctggtgtct    1680

ctatctgtag tactatgacc aatagacagt ttcatcaaaa attattgaaa tcaatagccg    1740

ccactagagg agctactgta gtaattggaa caagcaaatt ctatggtggt tggcacaaca 1800

tgttaaaaac tgtttatagt gatgtagaaa accctcacct tatgggttgg gattatccta 1860

aatgtgatag agccatgcct aacatgctta gaattatggc ctcacttgtt cttgctcgca 1920

aacatacaac gtgttgtagc ttgtcacacc gtttctatag attagctaat gagtgtgctc 1980

aagtattgag tgaaatggtc atgtgtggcg gttcactata tgttaaacca ggtggaacct 2040

catcaggaga tgccacaact gcttatgcta atagtgtttt taacatttgt caagctgtca 2100

cggccaatgt taatgcactt ttatctactg atggtaacaa aattgccgat aagtatgtcc 2160

gcaatttaca acacagactt tatgagtgtc tctatagaaa tagagatgtt gacacagact 2220

ttgtgaatga gttttacgca tatttgcgta aacatttctc aatgatgata ctctctgacg 2280

atgctgttgt gtgtttcaat agcacttatg catctcaagg tctagtggct agcataaaga 2340

actttaagtc agttctttat tatcaaaaca atgtttttat gtctgaagca aaatgttgga 2400

ctgagactga ccttactaaa ggacctcatg aattttgctc tcaacataca atgctagtta 2460

aacagggtga tgattatgtg taccttcctt acccagatcc atcaagaatc ctaggggccg 2520

gctgttttgt agatgatatc gtaaaaacag atggtacact tatgattgaa cggttcgtgt 2580

ctttagctat agatgcttac ccacttacta aacatcctaa tcaggagtat gctgatgtct 2640

ttcatttgta cttacaatac ataagaaagc tacatgatga gttaacagga cacatgttag 2700

acatgtattc tgttatgctt actaatgata acacttcaag gtattgggaa cctgagtttt 2760

atgaggctat gtacacaccg catacagtct tacag 2795

<210> 3
<211> 18
<212> DNA
<213> artificial sequence

<220>
<223> synthetic oligonucleotide

<400> 3
tcctgctaac aatgctgc 18

<210> 4
<211> 20
<212> DNA
<213> artificial sequence

<220>
<223> synthetic oligonucleotide

<400> 4
tctcaagctg gttcaatctg 20

<210> 5
<211> 40

<212>  DNA
<213>  artificial sequence

<220>
<223>  synthetic oligonucleotide

<400>  5
aacgagaaga ggcttgactg ctcaaggaac aacattgcca                              40


<210>  6
<211>  40
<212>  DNA
<213>  artificial sequence

<220>
<223>  synthetic oligonucleotide

<400>  6
ctcatcacgt agtcgcaaca gtattgccag ccattctagc                              40


<210>  7
<211>  19
<212>  DNA
<213>  artificial sequence

<220>
<223>  synthetic oligonucleotide

<400>  7
ccttctgcgt agaagcctt                                                     19


<210>  8
<211>  19
<212>  DNA
<213>  artificial sequence

<220>
<223>  synthetic oligonucleotide

<400>  8
aattcaactc caggcagca                                                     19


<210>  9
<211>  19
<212>  DNA
<213>  artificial sequence

<220>
<223>  synthetic oligonucleotide

<400>  9
tcaaggaaca acattgcca                                                     19


<210>  10
<211>  21
<212>  DNA
<213>  artificial sequence

<220>
<223>  synthetic oligonucleotide

<400>  10
aacgagaaga ggcttgactg c                                                21


<210>  11
<211>  18
<212>  DNA
<213>  artificial sequence

<220>
<223>  synthetic oligonucleotide

<400>  11
attgccagcc attctagc                                                    18


<210>  12
<211>  22
<212>  DNA
<213>  artificial sequence

<220>
<223>  synthetic oligonucleotide

<400>  12
ctcatcacgt agtcgcaaca gt                                               22


<210>  13
<211>  154
<212>  DNA
<213>  artificial sequence

<220>
<223>  synthetic oligonucleotide

<400>  13
tcaaggaaca acattgccaa aaggcttcta cgcagaaggg agcagaggcg gcagtcaagc      60

ctcttctcgt tcctcatcac gtagtcgcaa cagttcaaga aattcaactc caggcagcag     120

taggggaact tctcctgcta gaatggctgg caat                                 154


<210>  14
<211>  19
<212>  DNA
<213>  artificial sequence

<220>
<223>  synthetic oligonucleotide

<400>  14
tcaaggaaca acattgcca                                                   19


<210>  15
<211>  20
<212>  DNA
<213>  artificial sequence

<220>
<223>  synthetic oligonucleotide

<400>  15
cttcttagaa gcctcagcag                                                    20


<210>  16
<211>  38
<212>  DNA
<213>  artificial sequence

<220>
<223>  synthetic oligonucleotide

<400>  16
gccagccatt ctagcaggag cacgtagtcg caacagtt                                38


<210>  17
<211>  38
<212>  DNA
<213>  artificial sequence

<220>
<223>  synthetic oligonucleotide

<400>  17
aatggcggtg atgctgcttc tcaagctggt tcaatctg                                38


<210>  18
<211>  19
<212>  DNA
<213>  artificial sequence

<220>
<223>  synthetic oligonucleotide

<400>  18
tgctgcctgg agttgaatt                                                     19


<210>  19
<211>  18
<212>  DNA
<213>  artificial sequence

<220>
<223>  synthetic oligonucleotide

<400>  19
ttgctttgct gctgcttg                                                      18


<210>  20
<211>  18
<212>  DNA
<213>  artificial sequence

<220>
<223>  synthetic oligonucleotide

<400>  20
cacgtagtcg caacagtt                                                     18


<210>  21
<211>  20
<212>  DNA
<213>  artificial sequence

<220>
<223>  synthetic oligonucleotide

<400>  21
gccagccatt ctagcaggag                                                   20


<210>  22
<211>  20
<212>  DNA
<213>  artificial sequence

<220>
<223>  synthetic oligonucleotide

<400>  22
tctcaagctg gttcaatctg                                                   20


<210>  23
<211>  18
<212>  DNA
<213>  artificial sequence

<220>
<223>  synthetic oligonucleotide

<400>  23
aatggcggtg atgctgct                                                     18


<210>  24
<211>  132
<212>  DNA
<213>  artificial sequence

<220>
<223>  synthetic oligonucleotide

<400>  24
cacgtagtcg caacagttca agaaattcaa ctccaggcag cagtagggga acttctcctg      60

ctagaatggc tggcaatggc ggtgatgctg ctcttgcttt gctgctgctt gacagattga     120

accagcttga ga                                                          132


<210>  25
<211>  19
<212>  DNA
<213>  artificial sequence

<220>

<223>  synthetic oligonucleotide

<400>  25
tcaaggaaca acattgcca                                                    19


<210>  26
<211>  20
<212>  DNA
<213>  artificial sequence

<220>
<223>  synthetic oligonucleotide

<400>  26
cttcttagaa gcctcagcag                                                   20


<210>  27
<211>  38
<212>  DNA
<213>  artificial sequence

<220>
<223>  synthetic oligonucleotide

<400>  27
gccagccatt ctagcaggag cgtagtcgca acagttca                               38


<210>  28
<211>  38
<212>  DNA
<213>  artificial sequence

<220>
<223>  synthetic oligonucleotide

<400>  28
aatggcggtg atgctgcttc tcaagctggt tcaatctg                               38


<210>  29
<211>  19
<212>  DNA
<213>  artificial sequence

<220>
<223>  synthetic oligonucleotide

<400>  29
tgctgcctgg agttgaatt                                                    19


<210>  30
<211>  18
<212>  DNA
<213>  artificial sequence

<220>
<223>  synthetic oligonucleotide

<400>  30

ttgctttgct gctgcttg                                                    18


<210>  31
<211>  18
<212>  DNA
<213>  artificial sequence

<220>
<223>  synthetic oligonucleotide

<400>  31
cgtagtcgca acagttca                                                    18


<210>  32
<211>  20
<212>  DNA
<213>  artificial sequence

<220>
<223>  synthetic oligonucleotide

<400>  32
gccagccatt ctagcaggag                                                  20


<210>  33
<211>  20
<212>  DNA
<213>  artificial sequence

<220>
<223>  synthetic oligonucleotide

<400>  33
tctcaagctg gttcaatctg                                                  20


<210>  34
<211>  18
<212>  DNA
<213>  artificial sequence

<220>
<223>  synthetic oligonucleotide

<400>  34
aatggcggtg atgctgct                                                    18


<210>  35
<211>  130
<212>  DNA
<213>  artificial sequence

<220>
<223>  synthetic oligonucleotide

<400>  35
cgtagtcgca acagttcaag aaattcaact ccaggcagca gtaggggaac ttctcctgct      60

agaatggctg gcaatggcgg tgatgctgct cttgctttgc tgctgcttga cagattgaac     120

cagcttgaga                                                            130


<210>  36
<211>  20
<212>  DNA
<213>  artificial sequence

<220>
<223>  synthetic oligonucleotide

<400>  36
aatcgtgcta caacttcctc                                                  20


<210>  37
<211>  21
<212>  DNA
<213>  artificial sequence

<220>
<223>  synthetic oligonucleotide

<400>  37
ctcagcagca gatttcttag t                                                21


<210>  38
<211>  38
<212>  DNA
<213>  artificial sequence

<220>
<223>  synthetic oligonucleotide

<400>  38
cctactgctg cctggagttg agtcaagcct cttctcgt                              38


<210>  39
<211>  39
<212>  DNA
<213>  artificial sequence

<220>
<223>  synthetic oligonucleotide

<400>  39
ctcctgctag aatggctggc tcaatctgtc aagcagcag                             39


<210>  40
<211>  19
<212>  DNA
<213>  artificial sequence

<220>
<223>  synthetic oligonucleotide

<400>  40
ttgcgactac gtgatgagg                                                   19

<210>  41
<211>  18
<212>  DNA
<213>  artificial sequence

<220>
<223>  synthetic oligonucleotide

<400>  41
ggtgatgctg ctcttgct                                                    18


<210>  42
<211>  18
<212>  DNA
<213>  artificial sequence

<220>
<223>  synthetic oligonucleotide

<400>  42
agtcaagcct cttctcgt                                                    18


<210>  43
<211>  20
<212>  DNA
<213>  artificial sequence

<220>
<223>  synthetic oligonucleotide

<400>  43
cctactgctg cctggagttg                                                  20


<210>  44
<211>  19
<212>  DNA
<213>  artificial sequence

<220>
<223>  synthetic oligonucleotide

<400>  44
tcaatctgtc aagcagcag                                                   19


<210>  45
<211>  20
<212>  DNA
<213>  artificial sequence

<220>
<223>  synthetic oligonucleotide

<400>  45
ctcctgctag aatggctggc                                                  20


<210>  46
<211>  145

<212>  DNA
<213>  artificial sequence

<220>
<223>  synthetic oligonucleotide

<400>  46
agtcaagcct cttctcgttc ctcatcacgt agtcgcaaca gttcaagaaa ttcaactcca      60

ggcagcagta ggggaacttc tcctgctaga atggctggca atggcggtga tgctgctctt      120

gctttgctgc tgcttgacag attga                                            145


<210>  47
<211>  20
<212>  DNA
<213>  artificial sequence

<220>
<223>  synthetic oligonucleotide

<400>  47
tcaaggaaca acattgccaa                                                  20


<210>  48
<211>  20
<212>  DNA
<213>  artificial sequence

<220>
<223>  synthetic oligonucleotide

<400>  48
cttcttagaa gcctcagcag                                                  20


<210>  49
<211>  40
<212>  DNA
<213>  artificial sequence

<220>
<223>  synthetic oligonucleotide

<400>  49
gccagccatt ctagcaggag gtagtcgcaa cagttcaaga                            40


<210>  50
<211>  38
<212>  DNA
<213>  artificial sequence

<220>
<223>  synthetic oligonucleotide

<400>  50
aatggcggtg atgctgcttc tcaagctggt tcaatctg                              38


<210>  51

<211>  19
<212>  DNA
<213>  artificial sequence

<220>
<223>  synthetic oligonucleotide

<400>  51
tgctgcctgg agttgaatt                                                    19


<210>  52
<211>  18
<212>  DNA
<213>  artificial sequence

<220>
<223>  synthetic oligonucleotide

<400>  52
ttgctttgct gctgcttg                                                     18


<210>  53
<211>  20
<212>  DNA
<213>  artificial sequence

<220>
<223>  synthetic oligonucleotide

<400>  53
gtagtcgcaa cagttcaaga                                                   20


<210>  54
<211>  20
<212>  DNA
<213>  artificial sequence

<220>
<223>  synthetic oligonucleotide

<400>  54
gccagccatt ctagcaggag                                                   20


<210>  55
<211>  20
<212>  DNA
<213>  artificial sequence

<220>
<223>  synthetic oligonucleotide

<400>  55
tctcaagctg gttcaatctg                                                   20


<210>  56
<211>  18
<212>  DNA
<213>  artificial sequence

<220>
<223>  synthetic oligonucleotide

<400>  56
aatggcggtg atgctgct                                                    18


<210>  57
<211>  129
<212>  DNA
<213>  artificial sequence

<220>
<223>  synthetic oligonucleotide

<400>  57
gtagtcgcaa cagttcaaga aattcaactc caggcagcag taggggaact tctcctgcta      60

gaatggctgg caatggcggt gatgctgctc ttgctttgct gctgcttgac agattgaacc     120

agcttgaga                                                             129


<210>  58
<211>  18
<212>  DNA
<213>  artificial sequence

<220>
<223>  synthetic oligonucleotide

<400>  58
agtcaagcct cttctcgt                                                    18


<210>  59
<211>  18
<212>  DNA
<213>  artificial sequence

<220>
<223>  synthetic oligonucleotide

<400>  59
tgccgaggct tcttagaa                                                    18


<210>  60
<211>  36
<212>  DNA
<213>  artificial sequence

<220>
<223>  synthetic oligonucleotide

<400>  60
agcagcatca ccgccattaa ttcaactcca ggcagc                                36


<210>  61
<211>  39
<212>  DNA

<213>  artificial sequence

<220>
<223>  synthetic oligonucleotide

<400>  61
gctttgctgc tgcttgacag ttgttggcct ttaccagac                              39


<210>  62
<211>  19
<212>  DNA
<213>  artificial sequence

<220>
<223>  synthetic oligonucleotide

<400>  62
ccagccattc tagcaggag                                                    19


<210>  63
<211>  20
<212>  DNA
<213>  artificial sequence

<220>
<223>  synthetic oligonucleotide

<400>  63
attgaaccag cttgagagca                                                   20


<210>  64
<211>  18
<212>  DNA
<213>  artificial sequence

<220>
<223>  synthetic oligonucleotide

<400>  64
aattcaactc caggcagc                                                     18


<210>  65
<211>  18
<212>  DNA
<213>  artificial sequence

<220>
<223>  synthetic oligonucleotide

<400>  65
agcagcatca ccgccatt                                                     18


<210>  66
<211>  19
<212>  DNA
<213>  artificial sequence

<220>

<223>  synthetic oligonucleotide

<400>  66
ttgttggcct ttaccagac                                                    19


<210>  67
<211>  20
<212>  DNA
<213>  artificial sequence

<220>
<223>  synthetic oligonucleotide

<400>  67
gctttgctgc tgcttgacag                                                   20


<210>  68
<211>  135
<212>  DNA
<213>  artificial sequence

<220>
<223>  synthetic oligonucleotide

<400>  68
aattcaactc caggcagcag taggggaact tctcctgcta gaatggctgg caatggcggt      60

gatgctgctc ttgctttgct gctgcttgac agattgaacc agcttgagag caaaatgtct     120

ggtaaaggcc aacaa                                                       135


<210>  69
<211>  19
<212>  DNA
<213>  artificial sequence

<220>
<223>  synthetic oligonucleotide

<400>  69
ggcagtaacc agaatggag                                                    19


<210>  70
<211>  22
<212>  DNA
<213>  artificial sequence

<220>
<223>  synthetic oligonucleotide

<400>  70
aataccatct tggactgaga tc                                                22


<210>  71
<211>  38
<212>  DNA
<213>  artificial sequence

<220>
<223>  synthetic oligonucleotide

<400>  71
ttggaacgcc ttgtcctcga tactgcgtct tggttcac                              38


<210>  72
<211>  40
<212>  DNA
<213>  artificial sequence

<220>
<223>  synthetic oligonucleotide

<400>  72
accaatagca gtccagatga cccgtcacca ccacgaattc                            40


<210>  73
<211>  20
<212>  DNA
<213>  artificial sequence

<220>
<223>  synthetic oligonucleotide

<400>  73
ttccttgcca tgttgagtga                                                  20


<210>  74
<211>  21
<212>  DNA
<213>  artificial sequence

<220>
<223>  synthetic oligonucleotide

<400>  74
aattggctac taccgaagag c                                                21


<210>  75
<211>  19
<212>  DNA
<213>  artificial sequence

<220>
<223>  synthetic oligonucleotide

<400>  75
atactgcgtc ttggttcac                                                   19


<210>  76
<211>  19
<212>  DNA
<213>  artificial sequence

<220>
<223>  synthetic oligonucleotide

<400>  76
ttggaacgcc ttgtcctcg                                                    19


<210>  77
<211>  18
<212>  DNA
<213>  artificial sequence

<220>
<223>  synthetic oligonucleotide

<400>  77
cgtcaccacc acgaattc                                                     18


<210>  78
<211>  22
<212>  DNA
<213>  artificial sequence

<220>
<223>  synthetic oligonucleotide

<400>  78
accaatagca gtccagatga cc                                                22


<210>  79
<211>  153
<212>  DNA
<213>  artificial sequence

<220>
<223>  synthetic oligonucleotide

<400>  79
atactgcgtc ttggttcacc gctctcactc aacatggcaa ggaagacctt aaattccctc      60

gaggacaagg cgttccaatt aacaccaata gcagtccaga tgaccaaatt ggctactacc     120

gaagagctac cagacgaatt cgtggtggtg acg                                   153


<210>  80
<211>  20
<212>  DNA
<213>  artificial sequence

<220>
<223>  synthetic oligonucleotide

<400>  80
caaggcgttc caattaacac                                                   20


<210>  81
<211>  18
<212>  DNA
<213>  artificial sequence

<220>
<223>  synthetic oligonucleotide

<400>  81
gcagcattgt tagcagga                                                    18


<210>  82
<211>  40
<212>  DNA
<213>  artificial sequence

<220>
<223>  synthetic oligonucleotide

<400>  82
accgtcacca ccacgaattc caatagcagt ccagatgacc                            40


<210>  83
<211>  38
<212>  DNA
<213>  artificial sequence

<220>
<223>  synthetic oligonucleotide

<400>  83
ctaggaactg ggccagaagc acccatatga tgccgtct                              38


<210>  84
<211>  21
<212>  DNA
<213>  artificial sequence

<220>
<223>  synthetic oligonucleotide

<400>  84
gctcttcggt agtagccaat t                                                21


<210>  85
<211>  22
<212>  DNA
<213>  artificial sequence

<220>
<223>  synthetic oligonucleotide

<400>  85
gacttcccta tggtgctaac aa                                               22


<210>  86
<211>  20
<212>  DNA
<213>  artificial sequence

<220>
<223>  synthetic oligonucleotide

<400>  86
caatagcagt ccagatgacc                                                  20

<210>  87
<211>  20
<212>  DNA
<213>  artificial sequence

<220>
<223>  synthetic oligonucleotide

<400>  87
accgtcacca ccacgaattc                                                   20


<210>  88
<211>  18
<212>  DNA
<213>  artificial sequence

<220>
<223>  synthetic oligonucleotide

<400>  88
acccatatga tgccgtct                                                     18


<210>  89
<211>  20
<212>  DNA
<213>  artificial sequence

<220>
<223>  synthetic oligonucleotide

<400>  89
ctaggaactg ggccagaagc                                                   20


<210>  90
<211>  171
<212>  DNA
<213>  artificial sequence

<220>
<223>  synthetic oligonucleotide

<400>  90
caatagcagt ccagatgacc aaattggcta ctaccgaaga gctaccagac gaattcgtgg      60

tggtgacggt aaaatgaaag atctcagtcc aagatggtat ttctactacc taggaactgg     120

gccagaagct ggacttccct atggtgctaa caaagacggc atcatatggg t              171


<210>  91
<211>  19
<212>  DNA
<213>  artificial sequence

<220>
<223>  synthetic oligonucleotide

<400>  91

tcaacatggc aaggaagac                                                    19

<210>  92
<211>  18
<212>  DNA
<213>  artificial sequence

<220>
<223>  synthetic oligonucleotide

<400>  92
gcagcattgt tagcagga                                                     18

<210>  93
<211>  41
<212>  DNA
<213>  artificial sequence

<220>
<223>  synthetic oligonucleotide

<400>  93
accgtcacca ccacgaattc ttaacaccaa tagcagtcca g                           41

<210>  94
<211>  38
<212>  DNA
<213>  artificial sequence

<220>
<223>  synthetic oligonucleotide

<400>  94
ctaggaactg ggccagaagc acccatatga tgccgtct                               38

<210>  95
<211>  21
<212>  DNA
<213>  artificial sequence

<220>
<223>  synthetic oligonucleotide

<400>  95
gctcttcggt agtagccaat t                                                 21

<210>  96
<211>  22
<212>  DNA
<213>  artificial sequence

<220>
<223>  synthetic oligonucleotide

<400>  96
gacttcccta tggtgctaac aa                                                22

<210>  97
<211>  21
<212>  DNA
<213>  artificial sequence

<220>
<223>  synthetic oligonucleotide

<400>  97
ttaacaccaa tagcagtcca g                                                21


<210>  98
<211>  20
<212>  DNA
<213>  artificial sequence

<220>
<223>  synthetic oligonucleotide

<400>  98
accgtcacca ccacgaattc                                                  20


<210>  99
<211>  18
<212>  DNA
<213>  artificial sequence

<220>
<223>  synthetic oligonucleotide

<400>  99
acccatatga tgccgtct                                                    18


<210>  100
<211>  20
<212>  DNA
<213>  artificial sequence

<220>
<223>  synthetic oligonucleotide

<400>  100
ctaggaactg ggccagaagc                                                  20


<210>  101
<211>  178
<212>  DNA
<213>  artificial sequence

<220>
<223>  synthetic oligonucleotide

<400>  101
ttaacaccaa tagcagtcca gatgaccaaa ttggctacta ccgaagagct accagacgaa      60

ttcgtggtgg tgacggtaaa atgaaagatc tcagtccaag atggtatttc tactacctag     120

gaactgggcc agaagctgga cttccctatg gtgctaacaa agacggcatc atatgggt       178

<210>  102
<211>  19
<212>  DNA
<213>  artificial sequence

<220>
<223>  synthetic oligonucleotide

<400>  102
tcaacatggc aaggaagac                                                    19


<210>  103
<211>  18
<212>  DNA
<213>  artificial sequence

<220>
<223>  synthetic oligonucleotide

<400>  103
gcagcattgt tagcagga                                                     18


<210>  104
<211>  40
<212>  DNA
<213>  artificial sequence

<220>
<223>  synthetic oligonucleotide

<400>  104
accgtcacca ccacgaattc ccaatagcag tccagatgac                             40


<210>  105
<211>  38
<212>  DNA
<213>  artificial sequence

<220>
<223>  synthetic oligonucleotide

<400>  105
ctaggaactg ggccagaagc acccatatga tgccgtct                               38


<210>  106
<211>  21
<212>  DNA
<213>  artificial sequence

<220>
<223>  synthetic oligonucleotide

<400>  106
gctcttcggt agtagccaat t                                                 21


<210>  107
<211>  22

<212>  DNA
<213>  artificial sequence

<220>
<223>  synthetic oligonucleotide

<400>  107
gacttcccta tggtgctaac aa                                                22


<210>  108
<211>  20
<212>  DNA
<213>  artificial sequence

<220>
<223>  synthetic oligonucleotide

<400>  108
ccaatagcag tccagatgac                                                   20


<210>  109
<211>  20
<212>  DNA
<213>  artificial sequence

<220>
<223>  synthetic oligonucleotide

<400>  109
accgtcacca ccacgaattc                                                   20


<210>  110
<211>  18
<212>  DNA
<213>  artificial sequence

<220>
<223>  synthetic oligonucleotide

<400>  110
acccatatga tgccgtct                                                     18


<210>  111
<211>  20
<212>  DNA
<213>  artificial sequence

<220>
<223>  synthetic oligonucleotide

<400>  111
ctaggaactg ggccagaagc                                                   20


<210>  112
<211>  172
<212>  DNA
<213>  artificial sequence

<220>
<223>  synthetic oligonucleotide

<400>  112
ccaatagcag tccagatgac caaattggct actaccgaag agctaccaga cgaattcgtg      60

gtggtgacgg taaaatgaaa gatctcagtc caagatggta tttctactac ctaggaactg     120

ggccagaagc tggacttccc tatggtgcta acaaagacgg catcatatgg gt             172


<210>  113
<211>  20
<212>  DNA
<213>  artificial sequence

<220>
<223>  synthetic oligonucleotide

<400>  113
caaggcgttc caattaacac                                                  20


<210>  114
<211>  18
<212>  DNA
<213>  artificial sequence

<220>
<223>  synthetic oligonucleotide

<400>  114
gcagcattgt tagcagga                                                    18


<210>  115
<211>  39
<212>  DNA
<213>  artificial sequence

<220>
<223>  synthetic oligonucleotide

<400>  115
accgtcacca ccacgaattc atagcagtcc agatgacca                             39


<210>  116
<211>  38
<212>  DNA
<213>  artificial sequence

<220>
<223>  synthetic oligonucleotide

<400>  116
ctaggaactg ggccagaagc acccatatga tgccgtct                              38


<210>  117
<211>  21
<212>  DNA
<213>  artificial sequence

<220>
<223>  synthetic oligonucleotide

<400>  117
gctcttcggt agtagccaat t                                                21


<210>  118
<211>  22
<212>  DNA
<213>  artificial sequence

<220>
<223>  synthetic oligonucleotide

<400>  118
gacttcccta tggtgctaac aa                                               22


<210>  119
<211>  19
<212>  DNA
<213>  artificial sequence

<220>
<223>  synthetic oligonucleotide

<400>  119
atagcagtcc agatgacca                                                   19


<210>  120
<211>  20
<212>  DNA
<213>  artificial sequence

<220>
<223>  synthetic oligonucleotide

<400>  120
accgtcacca ccacgaattc                                                  20


<210>  121
<211>  18
<212>  DNA
<213>  artificial sequence

<220>
<223>  synthetic oligonucleotide

<400>  121
acccatatga tgccgtct                                                    18


<210>  122
<211>  20
<212>  DNA
<213>  artificial sequence

<220>
<223>  synthetic oligonucleotide

<400>  122
ctaggaactg ggccagaagc                                                   20


<210>  123
<211>  169
<212>  DNA
<213>  artificial sequence

<220>
<223>  synthetic oligonucleotide

<400>  123
atagcagtcc agatgaccaa attggctact accgaagagc taccagacga attcgtggtg       60

gtgacggtaa aatgaaagat ctcagtccaa gatggtattt ctactaccta ggaactgggc      120

cagaagctgg acttccctat ggtgctaaca aagacggcat catatgggt                  169


<210>  124
<211>  18
<212>  DNA
<213>  artificial sequence

<220>
<223>  synthetic oligonucleotide

<400>  124
tatggtggtt ggcacaac                                                     18


<210>  125
<211>  18
<212>  DNA
<213>  artificial sequence

<220>
<223>  synthetic oligonucleotide

<400>  125
tgtggcatct cctgatga                                                     18


<210>  126
<211>  43
<212>  DNA
<213>  artificial sequence

<220>
<223>  synthetic oligonucleotide

<400>  126
tgcgagcaag aacaagtgag gtgggttggg attatcctaa atg                         43


<210>  127
<211>  42
<212>  DNA
<213>  artificial sequence

<220>

<223>  synthetic oligonucleotide

<400>  127
tgttgtagct tgtcacaccg ttccacacat gaccatttca ct                          42


<210>  128
<211>  20
<212>  DNA
<213>  artificial sequence

<220>
<223>  synthetic oligonucleotide

<400>  128
taagcatgtt aggcatggct                                                   20


<210>  129
<211>  21
<212>  DNA
<213>  artificial sequence

<220>
<223>  synthetic oligonucleotide

<400>  129
agctaatgag tgtgctcaag t                                                 21


<210>  130
<211>  22
<212>  DNA
<213>  artificial sequence

<220>
<223>  synthetic oligonucleotide

<400>  130
tgggttggga ttatcctaaa tg                                                22


<210>  131
<211>  21
<212>  DNA
<213>  artificial sequence

<220>
<223>  synthetic oligonucleotide

<400>  131
tgcgagcaag aacaagtgag g                                                 21


<210>  132
<211>  20
<212>  DNA
<213>  artificial sequence

<220>
<223>  synthetic oligonucleotide

<400>  132

ccacacatga ccatttcact                                                    20


<210>  133
<211>  22
<212>  DNA
<213>  artificial sequence

<220>
<223>  synthetic oligonucleotide

<400>  133
tgttgtagct tgtcacaccg tt                                                  22


<210>  134
<211>  166
<212>  DNA
<213>  artificial sequence

<220>
<223>  synthetic oligonucleotide

<400>  134
tgggttggga ttatcctaaa tgtgatagag ccatgcctaa catgcttaga attatggcct        60

cacttgttct tgctcgcaaa catacaacgt gttgtagctt gtcacaccgt ttctatagat       120

tagctaatga gtgtgctcaa gtattgagtg aaatggtcat gtgtgg                      166


<210>  135
<211>  18
<212>  DNA
<213>  artificial sequence

<220>
<223>  synthetic oligonucleotide

<400>  135
tatggtggtt ggcacaac                                                       18


<210>  136
<211>  18
<212>  DNA
<213>  artificial sequence

<220>
<223>  synthetic oligonucleotide

<400>  136
tgtggcatct cctgatga                                                       18


<210>  137
<211>  43
<212>  DNA
<213>  artificial sequence

<220>
<223>  synthetic oligonucleotide

<400>  137
tgcgagcaag aacaagtgag ggggttggga ttatcctaaa tgt                    43


<210>  138
<211>  42
<212>  DNA
<213>  artificial sequence

<220>
<223>  synthetic oligonucleotide

<400>  138
tgttgtagct tgtcacaccg ttccacacat gaccatttca ct                    42


<210>  139
<211>  20
<212>  DNA
<213>  artificial sequence

<220>
<223>  synthetic oligonucleotide

<400>  139
taagcatgtt aggcatggct                                             20


<210>  140
<211>  21
<212>  DNA
<213>  artificial sequence

<220>
<223>  synthetic oligonucleotide

<400>  140
agctaatgag tgtgctcaag t                                           21


<210>  141
<211>  22
<212>  DNA
<213>  artificial sequence

<220>
<223>  synthetic oligonucleotide

<400>  141
gggttgggat tatcctaaat gt                                          22


<210>  142
<211>  21
<212>  DNA
<213>  artificial sequence

<220>
<223>  synthetic oligonucleotide

<400>  142
tgcgagcaag aacaagtgag g                                           21

<210>  143
<211>  20
<212>  DNA
<213>  artificial sequence

<220>
<223>  synthetic oligonucleotide

<400>  143
ccacacatga ccatttcact                                                    20


<210>  144
<211>  22
<212>  DNA
<213>  artificial sequence

<220>
<223>  synthetic oligonucleotide

<400>  144
tgttgtagct tgtcacaccg tt                                                 22


<210>  145
<211>  165
<212>  DNA
<213>  artificial sequence

<220>
<223>  synthetic oligonucleotide

<400>  145
gggttgggat tatcctaaat gtgatagagc catgcctaac atgcttagaa ttatggcctc        60

acttgttctt gctcgcaaac atacaacgtg ttgtagcttg tcacaccgtt tctatagatt       120

agctaatgag tgtgctcaag tattgagtga aatggtcatg tgtgg                       165


<210>  146
<211>  18
<212>  DNA
<213>  artificial sequence

<220>
<223>  synthetic oligonucleotide

<400>  146
gcctcacttg ttcttgct                                                      18


<210>  147
<211>  20
<212>  DNA
<213>  artificial sequence

<220>
<223>  synthetic oligonucleotide

<400>  147
cggacatact tatcggcaat                                                    20

<210>  148
<211>  38
<212>  DNA
<213>  artificial sequence

<220>
<223>  synthetic oligonucleotide

<400>  148
aaccgccaca catgaccatt ttgtagcttg tcacaccg                                38


<210>  149
<211>  39
<212>  DNA
<213>  artificial sequence

<220>
<223>  synthetic oligonucleotide

<400>  149
aggtggaacc tcatcaggag accgtgacag cttgacaaa                               39


<210>  150
<211>  22
<212>  DNA
<213>  artificial sequence

<220>
<223>  synthetic oligonucleotide

<400>  150
tcactcaata cttgagcaca ct                                                 22


<210>  151
<211>  20
<212>  DNA
<213>  artificial sequence

<220>
<223>  synthetic oligonucleotide

<400>  151
tgccacaact gcttatgcta                                                    20


<210>  152
<211>  18
<212>  DNA
<213>  artificial sequence

<220>
<223>  synthetic oligonucleotide

<400>  152
ttgtagcttg tcacaccg                                                      18


<210>  153

<211>  20
<212>  DNA
<213>  artificial sequence

<220>
<223>  synthetic oligonucleotide

<400>  153
aaccgccaca catgaccatt                                                    20


<210>  154
<211>  18
<212>  DNA
<213>  artificial sequence

<220>
<223>  synthetic oligonucleotide

<400>  154
ccgtgacagc ttgacaaa                                                      18


<210>  155
<211>  21
<212>  DNA
<213>  artificial sequence

<220>
<223>  synthetic oligonucleotide

<400>  155
aggtggaacc tcatcaggag a                                                  21


<210>  156
<211>  170
<212>  DNA
<213>  artificial sequence

<220>
<223>  synthetic oligonucleotide

<400>  156
ttgtagcttg tcacaccgtt tctatagatt agctaatgag tgtgctcaag tattgagtga        60

aatggtcatg tgtggcggtt cactatatgt taaaccaggt ggaacctcat caggagatgc       120

cacaactgct tatgctaata gtgtttttaa catttgtcaa gctgtcacgg                  170


<210>  157
<211>  18
<212>  DNA
<213>  artificial sequence

<220>
<223>  synthetic oligonucleotide

<400>  157
gcctcacttg ttcttgct                                                      18

<210>  158
<211>  20
<212>  DNA
<213>  artificial sequence

<220>
<223>  synthetic oligonucleotide

<400>  158
cggacatact tatcggcaat                                                    20


<210>  159
<211>  39
<212>  DNA
<213>  artificial sequence

<220>
<223>  synthetic oligonucleotide

<400>  159
ccgccacaca tgaccatttc tgttgtagct tgtcacacc                               39


<210>  160
<211>  39
<212>  DNA
<213>  artificial sequence

<220>
<223>  synthetic oligonucleotide

<400>  160
aggtggaacc tcatcaggag accgtgacag cttgacaaa                               39


<210>  161
<211>  22
<212>  DNA
<213>  artificial sequence

<220>
<223>  synthetic oligonucleotide

<400>  161
actcaatact tgagcacact ca                                                 22


<210>  162
<211>  20
<212>  DNA
<213>  artificial sequence

<220>
<223>  synthetic oligonucleotide

<400>  162
tgccacaact gcttatgcta                                                    20


<210>  163
<211>  19
<212>  DNA

<213>  artificial sequence

<220>
<223>  synthetic oligonucleotide

<400>  163
tgttgtagct tgtcacacc                                                    19


<210>  164
<211>  20
<212>  DNA
<213>  artificial sequence

<220>
<223>  synthetic oligonucleotide

<400>  164
ccgccacaca tgaccatttc                                                   20


<210>  165
<211>  18
<212>  DNA
<213>  artificial sequence

<220>
<223>  synthetic oligonucleotide

<400>  165
ccgtgacagc ttgacaaa                                                     18


<210>  166
<211>  21
<212>  DNA
<213>  artificial sequence

<220>
<223>  synthetic oligonucleotide

<400>  166
aggtggaacc tcatcaggag a                                                 21


<210>  167
<211>  172
<212>  DNA
<213>  artificial sequence

<220>
<223>  synthetic oligonucleotide

<400>  167
tgttgtagct tgtcacaccg tttctataga ttagctaatg agtgtgctca agtattgagt      60

gaaatggtca tgtgtggcgg ttcactatat gttaaaccag gtggaacctc atcaggagat     120

gccacaactg cttatgctaa tagtgttttt aacatttgtc aagctgtcac gg             172


<210>  168
<211>  21

<212>  DNA
<213>  artificial sequence

<220>
<223>  synthetic oligonucleotide

<400>  168
ccttatgggt tgggattatc c                                                21


<210>  169
<211>  19
<212>  DNA
<213>  artificial sequence

<220>
<223>  synthetic oligonucleotide

<400>  169
attaacattg gccgtgaca                                                   19


<210>  170
<211>  43
<212>  DNA
<213>  artificial sequence

<220>
<223>  synthetic oligonucleotide

<400>  170
aacggtgtga caagctacaa cagcttagaa ttatggcctc act                        43


<210>  171
<211>  39
<212>  DNA
<213>  artificial sequence

<220>
<223>  synthetic oligonucleotide

<400>  171
gtcatgtgtg gcggttcact aaagcagttg tggcatctc                             39


<210>  172
<211>  21
<212>  DNA
<213>  artificial sequence

<220>
<223>  synthetic oligonucleotide

<400>  172
gtatgtttgc gagcaagaac a                                                21


<210>  173
<211>  19
<212>  DNA
<213>  artificial sequence

<220>
<223>  synthetic oligonucleotide

<400>  173
ccaggtggaa cctcatcag                                                    19


<210>  174
<211>  21
<212>  DNA
<213>  artificial sequence

<220>
<223>  synthetic oligonucleotide

<400>  174
gcttagaatt atggcctcac t                                                 21


<210>  175
<211>  22
<212>  DNA
<213>  artificial sequence

<220>
<223>  synthetic oligonucleotide

<400>  175
aacggtgtga caagctacaa ca                                                22


<210>  176
<211>  18
<212>  DNA
<213>  artificial sequence

<220>
<223>  synthetic oligonucleotide

<400>  176
aagcagttgt ggcatctc                                                     18


<210>  177
<211>  21
<212>  DNA
<213>  artificial sequence

<220>
<223>  synthetic oligonucleotide

<400>  177
gtcatgtgtg gcggttcact a                                                 21


<210>  178
<211>  179
<212>  DNA
<213>  artificial sequence

<220>
<223>  synthetic oligonucleotide

<400>  178
gcttagaatt atggcctcac ttgttcttgc tcgcaaacat acaacgtgtt gtagcttgtc      60

acaccgtttc tatagattag ctaatgagtg tgctcaagta ttgagtgaaa tggtcatgtg     120

tggcggttca ctatatgtta aaccaggtgg aacctcatca ggagatgcca caactgctt      179


<210>  179
<211>  21
<212>  DNA
<213>  artificial sequence

<220>
<223>  synthetic oligonucleotide

<400>  179
ccttatgggt tgggattatc c                                                21


<210>  180
<211>  18
<212>  DNA
<213>  artificial sequence

<220>
<223>  synthetic oligonucleotide

<400>  180
tgtggcatct cctgatga                                                    18


<210>  181
<211>  42
<212>  DNA
<213>  artificial sequence

<220>
<223>  synthetic oligonucleotide

<400>  181
cacgttgtat gtttgcgagc aagtgataga gccatgccta ac                         42


<210>  182
<211>  42
<212>  DNA
<213>  artificial sequence

<220>
<223>  synthetic oligonucleotide

<400>  182
tgtagcttgt cacaccgttt ctccacacat gaccatttca ct                         42


<210>  183
<211>  22
<212>  DNA
<213>  artificial sequence

<220>
<223>  synthetic oligonucleotide

<400>  183
agtgaggcca taattctaag ca                                                22

<210>  184
<211>  21
<212>  DNA
<213>  artificial sequence

<220>
<223>  synthetic oligonucleotide

<400>  184
agctaatgag tgtgctcaag t                                                 21

<210>  185
<211>  20
<212>  DNA
<213>  artificial sequence

<220>
<223>  synthetic oligonucleotide

<400>  185
gtgatagagc catgcctaac                                                   20

<210>  186
<211>  22
<212>  DNA
<213>  artificial sequence

<220>
<223>  synthetic oligonucleotide

<400>  186
cacgttgtat gtttgcgagc aa                                                22

<210>  187
<211>  20
<212>  DNA
<213>  artificial sequence

<220>
<223>  synthetic oligonucleotide

<400>  187
ccacacatga ccatttcact                                                   20

<210>  188
<211>  22
<212>  DNA
<213>  artificial sequence

<220>
<223>  synthetic oligonucleotide

<400>  188
tgtagcttgt cacaccgttt ct                                                22

<210>  189
<211>  145
<212>  DNA
<213>  artificial sequence

<220>
<223>  synthetic oligonucleotide

<400>  189
gtgatagagc catgcctaac atgcttagaa ttatggcctc acttgttctt gctcgcaaac      60

atacaacgtg ttgtagcttg tcacaccgtt tctatagatt agctaatgag tgtgctcaag      120

tattgagtga aatggtcatg tgtgg                                            145


<210>  190
<211>  21
<212>  DNA
<213>  artificial sequence

<220>
<223>  synthetic oligonucleotide

<400>  190
ccttatgggt tgggattatc c                                                21


<210>  191
<211>  18
<212>  DNA
<213>  artificial sequence

<220>
<223>  synthetic oligonucleotide

<400>  191
tgtggcatct cctgatga                                                    18


<210>  192
<211>  41
<212>  DNA
<213>  artificial sequence

<220>
<223>  synthetic oligonucleotide

<400>  192
cacgttgtat gtttgcgagc aaaatgtgat agagccatgc c                          41


<210>  193
<211>  42
<212>  DNA
<213>  artificial sequence

<220>
<223>  synthetic oligonucleotide

<400>  193

tgtagcttgt cacaccgttt ctccacacat gaccatttca ct                42

<210>  194
<211>  22
<212>  DNA
<213>  artificial sequence

<220>
<223>  synthetic oligonucleotide

<400>  194
agtgaggcca taattctaag ca                                          22

<210>  195
<211>  21
<212>  DNA
<213>  artificial sequence

<220>
<223>  synthetic oligonucleotide

<400>  195
agctaatgag tgtgctcaag t                                           21

<210>  196
<211>  19
<212>  DNA
<213>  artificial sequence

<220>
<223>  synthetic oligonucleotide

<400>  196
aatgtgatag agccatgcc                                              19

<210>  197
<211>  22
<212>  DNA
<213>  artificial sequence

<220>
<223>  synthetic oligonucleotide

<400>  197
cacgttgtat gtttgcgagc aa                                          22

<210>  198
<211>  20
<212>  DNA
<213>  artificial sequence

<220>
<223>  synthetic oligonucleotide

<400>  198
ccacacatga ccatttcact                                             20

<210>  199
<211>  22
<212>  DNA
<213>  artificial sequence

<220>
<223>  synthetic oligonucleotide

<400>  199
tgtagcttgt cacaccgttt ct                                                22


<210>  200
<211>  148
<212>  DNA
<213>  artificial sequence

<220>
<223>  synthetic oligonucleotide

<400>  200
aatgtgatag agccatgcct aacatgctta gaattatggc ctcacttgtt cttgctcgca       60

aacatacaac gtgttgtagc ttgtcacacc gtttctatag attagctaat gagtgtgctc      120

aagtattgag tgaaatggtc atgtgtgg                                         148


<210>  201
<211>  21
<212>  DNA
<213>  artificial sequence

<220>
<223>  synthetic oligonucleotide

<400>  201
ccttatgggt tgggattatc c                                                 21


<210>  202
<211>  18
<212>  DNA
<213>  artificial sequence

<220>
<223>  synthetic oligonucleotide

<400>  202
tgtggcatct cctgatga                                                     18


<210>  203
<211>  42
<212>  DNA
<213>  artificial sequence

<220>
<223>  synthetic oligonucleotide

<400>  203
cacgttgtat gtttgcgagc aaatgtgata gagccatgcc ta                          42

<210>  204
<211>  42
<212>  DNA
<213>  artificial sequence

<220>
<223>  synthetic oligonucleotide

<400>  204
tgtagcttgt cacaccgttt ctccacacat gaccatttca ct                          42


<210>  205
<211>  22
<212>  DNA
<213>  artificial sequence

<220>
<223>  synthetic oligonucleotide

<400>  205
agtgaggcca taattctaag ca                                                22


<210>  206
<211>  21
<212>  DNA
<213>  artificial sequence

<220>
<223>  synthetic oligonucleotide

<400>  206
agctaatgag tgtgctcaag t                                                 21


<210>  207
<211>  20
<212>  DNA
<213>  artificial sequence

<220>
<223>  synthetic oligonucleotide

<400>  207
atgtgataga gccatgccta                                                   20


<210>  208
<211>  22
<212>  DNA
<213>  artificial sequence

<220>
<223>  synthetic oligonucleotide

<400>  208
cacgttgtat gtttgcgagc aa                                                22


<210>  209
<211>  20

<212>  DNA
<213>  artificial sequence

<220>
<223>  synthetic oligonucleotide

<400>  209
ccacacatga ccatttcact                                                   20


<210>  210
<211>  22
<212>  DNA
<213>  artificial sequence

<220>
<223>  synthetic oligonucleotide

<400>  210
tgtagcttgt cacaccgttt ct                                                22


<210>  211
<211>  147
<212>  DNA
<213>  artificial sequence

<220>
<223>  synthetic oligonucleotide

<400>  211
atgtgataga gccatgccta acatgcttag aattatggcc tcacttgttc ttgctcgcaa      60

acatacaacg tgttgtagct tgtcacaccg tttctataga ttagctaatg agtgtgctca     120

agtattgagt gaaatggtca tgtgtgg                                          147


<210>  212
<211>  22
<212>  DNA
<213>  artificial sequence

<220>
<223>  synthetic oligonucleotide

<400>  212
cacagttact tcacttcaga ct                                                22


<210>  213
<211>  18
<212>  DNA
<213>  artificial sequence

<220>
<223>  synthetic oligonucleotide

<400>  213
cgcagtaagg atggctag                                                     18


<210>  214

<211>  41
<212>  DNA
<213>  artificial sequence

<220>
<223>  synthetic oligonucleotide

<400>  214
gctagtagtc gtcgtcggtt ctgatgagcc tgaagaacat g                          41


<210>  215
<211>  44
<212>  DNA
<213>  artificial sequence

<220>
<223>  synthetic oligonucleotide

<400>  215
aagcacaagc tgatgagtac gagtaactag caagaatacc acga                       44


<210>  216
<211>  20
<212>  DNA
<213>  artificial sequence

<220>
<223>  synthetic oligonucleotide

<400>  216
ggatgaaccg tcgattgtgt                                                  20


<210>  217
<211>  19
<212>  DNA
<213>  artificial sequence

<220>
<223>  synthetic oligonucleotide

<400>  217
ttcggaagag acaggtacg                                                   19


<210>  218
<211>  20
<212>  DNA
<213>  artificial sequence

<220>
<223>  synthetic oligonucleotide

<400>  218
tgatgagcct gaagaacatg                                                  20


<210>  219
<211>  21
<212>  DNA
<213>  artificial sequence

<220>
<223>  synthetic oligonucleotide

<400>  219
gctagtagtc gtcgtcggtt c                                                21


<210>  220
<211>  22
<212>  DNA
<213>  artificial sequence

<220>
<223>  synthetic oligonucleotide

<400>  220
gtaactagca agaataccac ga                                               22


<210>  221
<211>  22
<212>  DNA
<213>  artificial sequence

<220>
<223>  synthetic oligonucleotide

<400>  221
aagcacaagc tgatgagtac ga                                               22


<210>  222
<211>  231
<212>  DNA
<213>  artificial sequence

<220>
<223>  synthetic oligonucleotide

<400>  222
tgatgagcct gaagaacatg tccaaattca cacaatcgac ggttcatccg gagttgttaa      60

tccagtaatg gaaccaattt atgatgaacc gacgacgact actagcgtgc ctttgtaagc     120

acaagctgat gagtacgaac ttatgtactc attcgtttcg gaagagacag gtacgttaat     180

agttaatagc gtacttcttt ttcttgcttt cgtggtattc ttgctagtta c              231


<210>  223
<211>  20
<212>  DNA
<213>  artificial sequence

<220>
<223>  synthetic oligonucleotide

<400>  223
agttccttgt tgtaacggtt                                                  20


<210>  224

<211>  20
<212>  DNA
<213>  artificial sequence

<220>
<223>  synthetic oligonucleotide

<400>  224
ctgctgaggc ttctaagaag                                                    20

## Description

### FIELD OF THE INVENTION

The invention relates to reagents and methods for the rapid detection of the presence and/or absence of SARS-CoV-2 and other betacoronavirus group B/C nucleic acid in a sample and their use for the diagnosis of an infection or the detection of an environmental contamination by SARS-CoV-2 and other betacoronavirus group B/C.

### BACKGROUND OF THE INVENTION

In 2019, a new coronavirus named SARS-CoV-2 which causes the disease COVID-19 was isolated in association with cases of severe acute respiratory syndrome named (Liu et al., Viruses, 2020 Jan 22;12(2)).

Coronaviruses are enveloped, positive-sense, single stranded RNA viruses that infect humans and mammals. The betacoronavirus genus (Beta-CoV or β-CoV) which is divided in 4 lineages or groups (A, B, C, D) comprise the highly human-pathogenic coronaviruses in groupB/C. Beta-CoV group B/C include SARS-Cov-2, SARS-Cov that emerged in China in 2002 (Group B) and the Middle East respiratory syndrome coronavirus (MERS-CoV), first detected in Saudi Arabia in 2012 (Group C; Zaki et al., N Engl J Med., 2012 Nov 8;367(19):1814-20 ; Lee et al., BMC Infect Dis. 2017 Jul 14;17(1):498). In contrast, Beta-CoV group A include HCoV-OC43 and HCoV-HKU1 which can cause the common cold.

SARS-Cov-2 genome comprises 6-11 open reading frames (ORFs) encoding a large polyprotein. The first ORF (ORF1a/b) which comprises a large portion of the genome encodes 16 nonstructural proteins (nsp) including papain-like protease (nsp3), RNA-dependent-RNA polymerase (RdRp or nsp12) and others likely to be involved in the transcription and replication of SARS-Cov-2. In addition to nsps, the genome encodes four major structural proteins including spike surface glycoprotein (S), membrane glycoprotein (M), nucleocapsid protein (N), envelope E and accessory protein like ORFs;

COVID-19 is difficult to differentiate clinically from the various etiologic agents of respiratory illnesses. Inexpensive, portable, and easy-to-use diagnostic devices are therefore urgently needed to ensure rapid detection of COVID-19 at the point-of-care. Unfortunately, the availability of laboratoty diagnosis in high disease-burden and low-resource areas is currently limited by cost, infrastructure, and personnel constraints. Rapid nucleic acid testing for viral diseases can provide access to much-needed diagnostic methods, especially for applications requiring fast turnaround times Among field-applicable isothermal nucleic acid amplification methods developed for rapid, simple, and cost-effective detection of pathogenic microorganisms in smaller size systems, the RT-LAMP (reverse-transcription loop-mediated isothermal amplification) appears among the most promising assays, highly suited for on-site detection of SARS-CoV-2 infections. The RT-LAMP assay uses a DNA polymerization enzyme with high strand-displacement activity and 6 primers, specifically designed to recognize 8 distinct regions on the target gene, to synthesize large amounts of target viral nucleic acids under a constant temperature (65°C). The accelerated RT-LAMP reaction yields high amount of amplification products, which can be detected in real time with simple detectors either by: (i) fluorescence, using DNA intercaling dyes; (ii) colorimetry, using pH indicators or (iii) turbidity, as the reaction produces large amounts of magnesium pyrophosphate (a white precipitate). In addition to its relative simplicity and low infrastructure costs, the RT-LAMP assay (i) has moderate incubation temperature leading to simplified heating and low power consumption, (ii) allows direct genetic amplification from target pathogens due to a superior tolerance to well-known PCR inhibitors such as sputum (iii) demonstrates high specificity and sensitivity and (iv) results in rapid detection often within 30 min.

There is an urgent need for robust RT-LAMP assays for the rapid detection of SARS-CoV-2. In addition, RT-LAMP assays allowing the differential diagnosis between infection and disease caused by SARS-CoV-2 and other highly human-pathogenic Beta-CoV group B/C (SARS-CoV, MERS-CoV) would be beneficial.

### SUMMARY OF THE INVENTION

The inventors have developed RT-LAMP primer sets targeting the N and RdRp genes which demonstrated a high analytical sensitivity with a limit of detection of 10 genome copies per reaction and a time-to result of less than 15 minutes. The amplification speed was increased or the limit-of-detection lower as compared to published primer sets (Lamb et al., PLOS ONE, 2020, June 12, 15:e0234682, doi: 10.1371; Yu et al., Clin. Chem., 2020 April 21, hvaa102.doi: 10.1093). Moreover, the optimized RT-LAMP primer sets demonstrated superior clinical sensitivity (100%) as compared to published primer set (50 %). This is surprising since the N gene which is a usual target of molecular diagnosis because of the abundance of its mRNA was previously reported as showing a poor sensitivity compared to other SARS-CoV-2 target genes such as Nsp3 (Park et al., The Journal of Molecular Diagnostics, 22, 6, June 2020). In addition, the combination of a SARS-CoV-2 specific RT-LAMP primer set (targeting the N gene) and a Beta-CoV group B/C specific RT-LAMP primer set (targeting the RdRp gene) provides a differential diagnosis between SARS-CoV-2 infection and disease and infection and disease caused by other Betacoronavirus group B/C such as SARS-CoV and MERS-CoV.

A first aspect of the invention relates to a set of oligonucleotide primers for amplification of Severe acute respiratory syndrome coronavirus 2 (SARS-CoV-2) RNA which comprises at least a forward primer and a reverse primer targeting SARS-CoV-2 Nucleocapsid (N) gene selected from the group consisting of:
- a forward primer which hybridizes to a sequence of at least 10 consecutive nucleotides, preferably at least 15 consecutive nucleotides from positions 486 to 505, 73 to 91, 171 to 189, 208 to 227, 450 to 467, 468 to 487, or 538 to 555 of said N gene complement sequence; and
- a reverse primer which hybridizes to a sequence of at least 10 consecutive nucleotides, preferably at least 15 consecutive nucleotides from positions 752 to 771, 307 to 328, 450 to 467, 450 to 467, 675 to 674, 739 to 759, or 762 to 779, respectively of said N gene sequence; and the indicated positions being determined by alignment with SEQ ID NO: 1.

In some embodiments, the first set of primers is for isothermal nucleic acid amplification of SARS-CoV-2 RNA and contains primers comprising sequences selected from the group consisting of: the sequences SEQ ID NO: 47 to 52 or variants thereof; the sequences SEQ ID NO: 3 to 8 or variants thereof; the sequences SEQ ID NO: 14 to 19 or variants thereof; the sequences SEQ ID NO: 25 to 30 or variants thereof; the sequences SEQ ID NO: 36 to 41 or variants thereof; the sequences SEQ ID NO: 58 to 63 or variants thereof; the sequences SEQ ID NO: 69 to 74 or variants thereof; the sequences SEQ ID NO: 80 to 85 or variants thereof; the sequences SEQ ID NO: 91 to 96 or variants thereof; the sequences SEQ ID NO: 102 to 107 or variants thereof; and the sequences SEQ ID NO: 113 to 118 or variants thereof.

In some embodiments, the first set of primers according to the present disclosure, which is specific for SARS-CoV-2.

Another aspect of the invention relates to a second set of oligonucleotide primers for amplification of Severe acute respiratory syndrome coronavirus 2 (SARS-CoV-2) RNA, which amplifies a target sequence of at least 100 nucleotides of SARS-CoV-2 RNA-dependent RNA polymerase (RdRp) gene situated between any one of positions 1819 to 1838 and any one of positions 2057 to 2076; any one of positions 1763 to 1782 and any one of positions 2057 to 2076; any one of positions 1819 to 1838 and any one of positions 2114 to 2133; and any one of positions 1880 to 1899 and any one of positions 2161 to 2180 of said RdRp gene sequence; and the indicated positions being determined by alignment with SEQ ID NO: 2.

In some embodiments, the second set of oligonucleotide primers, comprises at least a forward and a reverse primer targeting SARS-CoV-2 RNA-dependent RNA polymerase (RdRp) gene selected from the group consisting of:
- a forward primer which hybridizes to a sequence of at least 10 consecutive nucleotides, preferably at least 15 consecutive nucleotides from positions 1838 to 1858, 1782 to 1799, 1838 to 1858, or 1899 to 1916 of said RdRp gene complement sequence; and
- a reverse primer which hybridizes to a sequence of at least 10 consecutive nucleotides, preferably at least 15 consecutive nucleotides from positions 2040 to 2057, 2040 to 2057, 2096 to 2114, or 2142 to 2161, respectively of said RdRp gene sequence; and the indicated positions being determined by alignment with SEQ ID NO: 2.

In some particular embodiments, the second set of primers is for isothermal nucleic acid amplification of SARS-CoV-2 RNA and contains primers comprising sequences selected from the group consisting of: the sequences SEQ ID NO: 179 to 184 or variants thereof; the sequences SEQ ID NO: 124 to 129 or variants thereof; the sequences SEQ ID NO: 135 to 140 or variants thereof; the sequences SEQ ID NO: 146 to 151 or variants thereof; the sequences SEQ ID NO: 157 to 162 or variants thereof; the sequences SEQ ID NO: 168 to 173 or variants thereof; the sequences SEQ ID NO: 190 to 195 or variants thereof; and the sequences SEQ ID NO: 201 to 206 or variants thereof.

In some particular embodiments, the second set of primers according to according to the present disclosure, which is specific for Betacoronavirus group B/C.

In some particular embodiments, the first or second set of oligonucleotide primers according to the present disclosure, which comprise at least one labelled oligonucleotide primer; preferably a fluorescent oligonucleotide primer.

Another aspect of the invention relates to a combination of oligonucleotide primers comprising at least a first set of primers specific for SARS-CoV-2 according to the present disclosure and a second set of primers specific for Betacoronavirus group B/C.according to to the present disclosure.

In some particular embodiments, the combination contains a first set primers comprising sequences selected from the group consisting of the sequences SEQ ID NO: 47 to 52 or variants thereof and a second set of primers comprising sequences selected from the group consisting of the sequences SEQ ID NO: 179 to 184 or variants thereof.

In some particular embodiments, the combination provides a differential diagnosis between infection and disease caused by SARS-CoV-2 and other Betacoronavirus group B/C such as SARS-CoV and MERS-CoV.

Another aspect of the invention relates to a kit for the detection of SARS-CoV-2 and/or Betacoronavirus group B/C RNA, comprising at least one set of oligonucleotide primers or a combination thereof according to the present disclosure.

Another aspect of the invention relates to the use of the kit according to the present disclosure, for the *in vitro* diagnosis of SARS-CoV-2 and/or Betacoronavirus group B/C infection and associated disease in a biological sample from a subject.

Another aspect of the invention relates to the use of the kit according to according to the present disclosure for the *in vitro* detection of a contamination with SARS-CoV-2 and/or Betacoronavirus group B/C in an environmental sample.

Another aspect of the invention relates to a method of detection of SARS-CoV-2and/ or Betacoronavirus group B/C RNA in a sample, comprising :
- subjecting said sample to an isothermal nucleic acid amplification reaction using a set of primers according to according to the present disclosure for amplifying a target sequence of said viral RNA, and
- detecting the presence of an amplification product for said target sequence.

### DETAILED DESCRIPTION OF THE INVENTION

The invention relates to sets of oligonucleotide primers targeting the N or RdRp genes for rapid and sensitive amplification, in particular isothermal nucleic acid amplification, and detection of Severe acute respiratory syndrome coronavirus 2 (SARS-CoV-2) nucleic acid. The oligonucleotide primer sets are useful alone or in combination for the specific diagnosis of SARS-CoV-2 and Betacoronavirus group B/C infections in a biological sample or detection of SARS-CoV-2 and Betacoronavirus group B/C contaminations in an environmental sample. The invention encompasses the derived Kit and method for the detection or diagnostics of SARS-CoV-2 and Betacoronavirus group B/C comprising at least a set of oligonucleotide primers or combination thereof according to the present disclosure.

A first aspect of the invention relates to a first set of oligonucleotide primers for amplification of Severe acute respiratory syndrome coronavirus 2 (SARS-CoV-2) nucleic acid which comprises at least a forward primer and a reverse primer targeting SARS-CoV-2 Nucleocapsid (N) gene selected from the group consisting of:
- a forward primer which hybridizes to a sequence of at least 10 consecutive nucleotides, preferably at least 15 consecutive nucleotides from positions 486 to 505 of said N gene complement sequence and a reverse primer which hybridizes to a sequence of at least 10 consecutive nucleotides, preferably at least 15 consecutive nucleotides from positions 752 to 771 of said N gene sequence;
- a forward primer which hybridizes to a sequence of at least 10 consecutive nucleotides, preferably at least 15 consecutive nucleotides from positions 73 to 91 of said N gene complement sequence and a reverse primer which hybridizes to a sequence of at least 10 consecutive nucleotides, preferably at least 15 consecutive nucleotides from positions 307 to 328 of said N gene sequence;
- a forward primer which hybridizes to a sequence of at least 10 consecutive nucleotides, preferably at least 15 consecutive nucleotides from positions 171 to 189 of said N gene complement sequence and a reverse primer which hybridizes to a sequence of at least 10 consecutive nucleotides, preferably at least 15 consecutive nucleotides from positions 450 to 467 of said N gene sequence;
- a forward primer which hybridizes to a sequence of at least 10 consecutive nucleotides, preferably at least 15 consecutive nucleotides from positions 208 to 227 of said N gene complement sequence and a reverse primer which hybridizes to a sequence of at least 10 consecutive nucleotides, preferably at least 15 consecutive nucleotides from positions 450 to 467 of said N gene sequence;
- a forward primer which hybridizes to a sequence of at least 10 consecutive nucleotides, preferably at least 15 consecutive nucleotides from positions 450 to 467 of said N gene complement sequence and a reverse primer which hybridizes to a sequence of at least 10 consecutive nucleotides, preferably at least 15 consecutive nucleotides from positions 675 to 674 of said N gene sequence;
- a forward primer which hybridizes to a sequence of at least 10 consecutive nucleotides, preferably at least 15 consecutive nucleotides from positions 468 to 487 of said N gene complement sequence and a reverse primer which hybridizes to a sequence of at least 10 consecutive nucleotides, preferably at least 15 consecutive nucleotides from positions 739 to 759 of said N gene sequence; and
- a forward primer which hybridizes to a sequence of at least 10 consecutive nucleotides, preferably at least 15 consecutive nucleotides from positions 538 to 555 of said N gene sequence and a reverse primer which hybridizes to a sequence of at least 10 consecutive nucleotides, preferably at least 15 consecutive nucleotides from positions 762 to 779 of said N gene sequence; and the indicated positions being determined by alignment with SEQ ID NO: 1.

The Nucleocapsid (N) gene or ORF9 of SARS-CoV-2 (Gene ID: 43740575) corresponds to the nucleotide sequence from positions 28274 to 29533 of GenBank accession number NC_045512.2 accessed on March 30, 2020 or SEQ ID NO: 1 (1260 nt) which codes for the nucleocapsid phosphoprotein (GenPept accession number YP_009724397.2 accessed on March 30, 2020; 419 aa) comprising the Nucleocapsid (NP) protein from positions 14 to 368. SEQ ID NO : 1 is the sequence of the N gene from SARS-CoV-2 isolate Wuhan-Hu-1. One skilled in the art can easily determine the target sequence of the set of primers according to the invention in the N gene of any other SARS-CoV-2 isolate by alignment with SEQ ID NO: 1 using appropriate software available in the art such as BLAST, CLUSTALW and others.

As used herein "hybridize" refers to the ability to form a double-stranded hybrid molecule with SARS-CoV-2 nucleic acid (RNA, DNA equivalent or complement thereof) sufficient to produce a cDNA and to promote amplification of the cDNA in the conditions used for nucleic acid amplification such as those disclosed in the examples. The reverse primer is an anti-sense primer which hybridizes to SARS-CoV-2 RNA or DNA equivalent generated by amplification. The forward primer is a sense primer which hybridizes to SARS-CoV-2 cDNA (complementary DNA) or DNA copies thereof generated by amplification. All the sequences are provided in their 5' to 3" orientation. For example, the sequence from positions 486 to 505 of said N gene is 5'-TCAAGGAACAACATTGCCAA-3' (SEQ ID NO: 47); its complement is 5'-AGTTCCTTGTTGTAACGGTT-3' (SEQ ID NO: 223). The forward primer which hybridizes to at least 10 consecutive nucleotides, preferably at least 15 consecutive nucleotides from positions 486 to 505 of said N gene complement sequence is for example the forward primer 5'-TCAAGGAACAACATTGCCAA-3' (SEQ ID NO: 47). The sequence from positions 307 to 328 of said N gene sequence is 5'-CTGCTGAGGCTTCTAAGAAG-3' (SEQ ID NO: 224). The reverse primer which hybridizes to a sequence of at least 10 consecutive nucleotides, preferably at least 15 consecutive nucleotides from positions 307 to 328 of said N gene sequence is for example the primer 5'-CTTCTTAGAAGCCTCAGCAG-3' (SEQ ID NO: 48) which is the reverse-complement of SEQ ID NO: 224.

The set of primer according to the invention comprises a least a pair of primers (forward primer and reverse primer) and may comprise additional primers, in particular for isothermal nucleic amplification, the set of primers comprise 3 pairs of primers: External (F3 and B3); Internal (Forward inner primer or FIP(F1c+F2) and Backward inner primer or BIP (B1c+B2)); and Loop (Loop Forward or LoopF and Loop Backward (LoopB)).

Another aspect of the invention relates to a second set of oligonucleotide primers for amplification of Severe acute respiratory syndrome coronavirus 2 (SARS-CoV-2) nucleic acid which amplifies a target sequence of at least 100 nucleotides of SARS-CoV-2 RNA-dependent RNA polymerase (RdRp) gene situated between any one of positions 1819 to 1838 and any one of positions 2057 to 2076; any one of positions 1763 to 1782 and any one of positions 2057 to 2076; any one of positions 1819 to 1838 and any one of positions 2114 to 2133; and any one of positions 1880 to 1899 and any one of positions 2161 to 2180 of said RdRp gene sequence; and the indicated positions being determined by alignment with SEQ ID NO: 2.

The RNA-dependent RNA polymerase (RdRp) gene or nsp12 of SARS-CoV-2 corresponds to the sequence from positions 13442 to 13468 joined to positions 13468 to 16236 of the nucleotide sequence GenBank accession number NC_045512.2 accessed on March 30, 2020 or SEQ ID NO: 2 which codes for the RNA-dependent RNA polymerase (RdRp) protein (GenPept accession number YP_009725307.1 accessed on March 30, 2020 ; 932 aa). Positions 1782 to 1799; 2040 to 2057; 1838 to 1858; 2096 to 2114; 1899 to 1916 and 2142 to 2161 of SEQ ID NO: 2 correspond to positions 15223-15240, 15481-15498, 15279-15299, 15537-15555, 15340-15357, and 15583-15602 respectively of GenBank accession number NC_045512.2. The sequence SEQ ID NO: 2 is the sequence of the RdRp gene from SARS-CoV-2 isolate Wuhan-Hu-1. One skilled in the art can easily determine the target sequence of the set of primers according to the invention in the RdRp gene of any other SARS-CoV-2 isolate by alignment with SEQ ID NO: 2 or GenBank accession number NC_045512.2 using appropriate software available in the art such as BLAST, CLUSTALW and others.

The oligonucleotides according to the present disclosure which function as primers are capable of annealing specifically to a target sequence in the N or RdRp gene of SARS-CoV-2 nucleic acid and can be further extended in the presence of a nucleic acid polymerase to specifically amplify the target sequence. The target sequence refers to the particular nucleotide sequence of the target nucleic acid that is to be amplified and detected. The target sequence includes the sequences to which oligonucleotide primers hybridize during the nucleic acid amplification process. Where the target nucleic acid is originally single-stranded, the term target sequence will also refer to the sequence complementary to the target sequence as present in the target nucleic acid. Where the target nucleic acid is originally double-stranded the term target-sequence refers to both the sense (+) and antisense (-) strands. The target sequence consists of at least 50 consecutive nucleotides, preferably at least 100, 125 or 150 consecutive nucleotides from the target nucleic acid. Preferably, the target sequence consists of 100 to 350 consecutive nucleotides from the sequence of the target nucleic acid, more preferably 125 to 200 consecutive nucleotides. The target sequence is a unique sequence which is specific for SARS-CoV-2 or Betacoronavirus group B/C. Thus, the detection of the target sequence indicates the presence of SARS-CoV-2 or Betacoronavirus group B/C in the sample. Therefore, the detection of the target sequence in a biological sample is indicative of whether the individual is suffering from SARS-CoV-2 or Betacoronavirus group B/C virus infection and in particular from a disease caused by SARS-CoV-2 (Covid-19) or Betacoronavirus group B/C virus. The detection of the target sequence in an environmental sample is indicative of whether the environment is contaminated with SARS-CoV-2 or Betacoronavirus group B/C virus.

The target nucleic acid refers to a nucleic acid comprising a target sequence to be amplified. The target nucleic acid may include other sequences besides the target sequence, which may not be amplified. The viral nucleic acid or target nucleic acid may be genomic RNA (viral RNA or vRNA) or viral mRNA. In some preferred embodiments of the invention, the target nucleic acid is viral RNA (*i.e.* SARS-CoV-2 or Betacoronavirus group B/C genomic RNA).

Oligonucleotide refers to a polymer of 5 to 100 nucleotides, generally from a lower limit of 15 to 20 nucleotides to an upper limit of 45 to 60 nucleotides, preferably from about 15 to about 45 nucleotides, wherein the polymer comprise ribonucleotides, deoxyribonucleotides, modified nucleotides or mixtures thereof and may further include modified internucleotide linkages and/or modified 5' and/or 3' termini. Oligonucleotides are usually synthesized using any of a variety of well-known enzymatic or chemical methods. The oligonucleotide primer according to the invention is substantially complementary to the target sequence. Substantially complementary means that the oligonucleotide is at least 80% identical, preferably at least 85%, 90%, 95% and 98% identical to the target sequence. The oligonucleotide may comprise additional sequences (not complementary to the target sequence) at its 5' end. In some embodiments the oligonucleotide comprises a sequence of at least 5, preferably 10 to 15 consecutive nucleotides which is 100% identical to the target sequence. In some more preferred embodiments, the oligonucleotide sequence is 100% identical to the target sequence. Optionally, at least one oligonucleotide of the pair includes a label (detectable moiety).

At least one oligonucleotide may also function as a probe and further includes a detectable label to detect a target nucleic acid or an amplicon thereof. The detectable label is a moiety that can be detected directly or indirectly by the production of a detectable signal such as colorimetric, fluorescent, chemiluminescent or electrochemoluminescent signal. Directly detectable labels include radioisotopes and fluorophores. Indirectly detectable labels are detected by labeling with additional reagents that enable the detection. Indirectly detectable labels include, for example, chemiluminescent agents, enzymes that produce visible or colored reaction products, and a ligand-detectable ligand binding partner, where a ligand (hapten, antibody, antigen, biotin) may be detected by binding to a labelled ligand-specific binding partner.

The oligonucleotides are designed based on SARS-CoV-2 and other Coronavirus group B/C including genomic sequences available in sequence databases using general principles for designing amplification primers. The design of the primers may also include specific principles for designing primers for use in specific isothermal amplification methods. Primer design may be performed using any of the various softwares.

As used herein, the expression "percentage of identity" between two nucleic acid sequences, means the percentage of identical nucleic acid, between the two sequences to be compared, obtained with the best alignment of said sequences, this percentage being purely statistical and the differences between these two sequences being randomly spread over the nucleic acid acids sequences. As used herein, "best alignment" or "optimal alignment", means the alignment for which the determined percentage of identity (see below) is the highest. Sequence comparison between two nucleic acids sequences is usually realized by comparing these sequences that have been previously aligned according to the best alignment; this comparison is realized on segments of comparison in order to identify and compare the local regions of similarity. The best sequences alignment to perform comparison can be realized, besides manually, by using the global homology algorithm developed by SMITH and WATERMAN (Ad. App. Math., vol.2, p:482, 1981), by using the local homology algorithm developed by NEDDLEMAN and WUNSCH (J. Mol. Biol, vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C, Nucleic Acids Research, vol. 32, p: 1792, 2004). To get the best local alignment, one can preferably use BLAST software. The identity percentage between two sequences of nucleic acids is determined by comparing these two sequences optimally aligned, the nucleic acids sequences being able to comprise additions or deletions in respect to the reference sequence in order to get the optimal alignment between these two sequences. The percentage of identity is calculated by determining the number of identical positions between these two sequences, and dividing this number by the total number of compared positions, and by multiplying the result obtained by 100 to get the percentage of identity between these two sequences.

As used herein, "Individual", "subject" or "patient" refers to a human or an animal. An animal includes a mammal and others.

The terms "a", "an", and "the" include plural referents, unless the context clearly indicates otherwise. For example "a primer" as used herein is understood to represent one or more primers. As such, the term "a" (or "an"), "one or more" or "at least one" can be used interchangeably herein.

In some preferred embodiments, the first set of oligonucleotide primers comprises a forward primer which hybridizes to a sequence of at least 10 consecutive nucleotides, preferably at least 15 consecutive nucleotides from positions 486 to 505 of said N gene complement sequence and a reverse primer which hybridizes to a sequence of at least 10 consecutive nucleotides, preferably at least 15 consecutive nucleotides from positions 752 to 771 of said N gene sequence.

In some particular embodiments, the second set of oligonucleotide primers amplifies a target sequence of at least 100 nucleotides of SARS-CoV-2 RNA-dependent RNA polymerase (RdRp) gene situated between positions 1838 and 2057; 1782 and 2057; 1838 and 2114 ; and 1899 and 2161 of said RdRp gene sequence; and the indicated positions being determined by alignment with SEQ ID NO: 2.

In some particular embodiments, the oligonucleotide primers of the second set are selected from the group consisting of the sequences of 5 to 100 nucleotides, preferably 15 to 45 nucleotides having 80% to 100% identity, preferably at least 85%, 90% 95% or 98% identity with any of the sequence from positions 1833 to 2062; 1777 to 2062; 1833 to 2119 ; and 1894 to 2166 of said RdRp gene sequence or the complement thereof; preferably any one the sequence from positions 1838 to 2057; 1782 to 2057; 1838 to 2114 ; and 1899 to 2161 of said RdRp gene sequence or the complement thereof.

In some particular embodiments, the second set of oligonucleotide primers comprises at least a forward primer and a reverse primer targeting SARS-CoV-2 RNA-dependent RNA polymerase (RdRp) gene selected from the group consisting of:
- a forward primer which hybridizes to a sequence of at least 10 consecutive nucleotides, preferably at least 15 consecutive nucleotides from positions 1838 to 1858 of said RdRp gene complement sequence and a reverse primer which hybridizes to a sequence of at least 10 consecutive nucleotides, preferably at least 15 consecutive nucleotides from positions 2040 to 2057 of said RdRp gene sequence;
- a forward primer which hybridizes to a sequence of at least 10 consecutive nucleotides, preferably at least 15 consecutive nucleotides from positions 1782 to 1799 of said RdRp gene complement sequence and a reverse which hybridizes to a sequence of at least 10 consecutive nucleotides, preferably at least 15 consecutive nucleotides from positions 2040 to 2057 of said RdRp gene sequence;
- a forward primer which hybridizes to a sequence of at least 10 consecutive nucleotides, preferably at least 15 consecutive nucleotides from positions 1838 to 1858 of said RdRp gene complement sequence and a reverse primer which hybridizes to a sequence of at least 10 consecutive nucleotides, preferably at least 15 consecutive nucleotides from positions 2096 to 2114 of said RdRp gene sequence; and
- a forward primer which hybridizes to a sequence of at least 10 consecutive nucleotides, preferably at least 15 consecutive nucleotides from positions 1899 to 1916 of said RdRp gene complement sequence and a reverse primer which hybridizes to a sequence of at least 10 consecutive nucleotides, preferably at least 15 consecutive nucleotides from positions 2142 to 2161 of said RdRp gene sequence; and the indicated positions being determined by alignment with SEQ ID NO: 2.

In some particular embodiments, the first set of oligonucleotide primers is specific for SARS-CoV-2.

In some particular embodiments, the second set of oligonucleotide primers is specific for Betacoronavirus group B/C. Betacoronavirus group B/C includes human pathogenic coronaviruses such as SARS-CoV and SARS-CoV-2 (Group B) and MERS-CoV (Group C). Betacoronavirus group B/C are different from other Betacoronavirus groups such as in particular Betacoronavirus group A which include OC43 which can cause the common cold and HKU1.

In some preferred embodiments, the first set and/or the second set of oligonucleotide primers is for isothermal nucleic acid amplification of SARS-CoV-2 nucleic acid.

In some particular embodiments, the first set of primers for isothermal nucleic acid amplification of SARS-CoV-2 nucleic acid is selected from the group consisting of:
- a set E of primers comprising the sequences SEQ ID NO: 47 to 52 or variants thereof;
- a set A of primers comprising the sequences SEQ ID NO: 3 to 8 or variants thereof;
- a set B of primers comprising the sequences SEQ ID NO: 14 to 19 or variants thereof;
- a set C of primers comprising primers comprising the sequences SEQ ID NO: 25 to 30 or variants thereof;
- a set D of primers comprising the sequences SEQ ID NO: 36 to 41 or variants thereof;
- a set F of primers comprising the sequences SEQ ID NO: 58 to 63 or variants thereof;
- a set G of primers comprising the sequences SEQ ID NO: 69 to 74 or variants thereof;
- a set H of primers comprising the sequences SEQ ID NO: 80 to 85 or variants thereof;
- a set I of primers comprising the sequences SEQ ID NO: 91 to 96 or variants thereof;
- a set J of primers comprising the sequences SEQ ID NO: 102 to 107 or variants thereof; and
- a set K of primers comprising the sequences SEQ ID NO: 113 to 118 or variants thereof.

The primer may comprise or consist of the above-mentioned sequence. The External and Loop primers for isothermal nucleic acid amplification consist usually of 15 to 25 nucleotides, whereas the Internal primers consist of 35 to 45 nucleotides. The variants or sequence variants refer to functional primers which hybridize to SARS-CoV-2 nucleic acid (RNA, DNA equivalent or complement thereof), e.g., have the ability to form a double-stranded hybrid molecule with SARS-CoV-2 nucleic acid (RNA, DNA equivalent or complement thereof) sufficient to produce a cDNA and to promote amplification of the cDNA in the conditions used for nucleic acid amplification such as those disclosed in the examples. For primers of up to 20 nucleotides, the sequence variants may comprise up to 5 nucleotide differences (1, 2, 3, 4 or 5 nucleotide differences) with the parent sequence; for primers of more than 20 nucleotides, the sequence variants may comprise up to 10 nucleotide differences (1, 2, 3, 4, 5, 6, 7, 8, 9, 10 nucleotide differences) with the parent sequence, for examples SEQ ID 47 to 52 for set E. One nucleotide difference refers to the deletion, insertion or substitution of one nucleotide. In some embodiments, the sequence variant has at least 80% identity, preferably at least 85%, 90%, 95% and 98% identity with the parent sequence.

In some more preferred embodiments, the first set of oligonucleotide primers for isothermal nucleic acid amplification of SARS-CoV-2 nucleic is selected from the group consisting of: a set A, B, C, E, F or G; preferably a set A, B, E, or F, or a set C, E, F, or G; more preferably a set C, E, or G; still more preferably a set E as defined above.

In some particular embodiments, the second set of primers for isothermal nucleic acid amplification of SARS-CoV-2 nucleic acid is selected from the group consisting of:
- a set T of primers comprising the sequences SEQ ID NO: 179 to 184 or variants thereof;
- a set L of primers comprising the sequences SEQ ID NO: 124 to 129 or variants thereof;
- a set M of primers comprising the sequences SEQ ID NO: 135 to 140 or variants thereof;
- a set N of primers comprising the sequences SEQ ID NO: 146 to 151 or variants thereof;
- a set O of primers comprising the sequences SEQ ID NO: 157 to 162 or variants thereof;
- a set P of primers comprising the sequences SEQ ID NO: 168 to 173 or variants thereof;
- a set U of primers comprising the sequences SEQ ID NO: 190 to 195 or variants thereof; and
- a set V of primers comprising the sequences SEQ ID NO: 201 to 206 or variants thereof.

In some more preferred embodiments, the second set of oligonucleotide primers for isothermal nucleic acid amplification of SARS-CoV-2 nucleic acid is selected from the group consisting of: a set L, M, T, U or V; or a set N, O, P or T; preferably a set T, U or V; or a set N, P or T; more preferably a set T as defined above.

In some preferred embodiments, the first or second set of oligonucleotide primers, in particular a set of oligonucleotide primer for isothermal nucleic acid amplification of SARS-CoV-2 nucleic acid, comprise at least one labelled oligonucleotide primer; preferably a fluorescent oligonucleotide primer.

Another aspect of the invention relates to a combination of oligonucleotide primers comprising at least a first set and a second set of oligonucleotide primers according to the present disclosure. In some particular embodiments, the combination comprises a first set of oligonucleotide primers which is specific for SARS-CoV-2 and a second set of oligonucleotide primers which is specific for Betacoronavirus group B/C. In some preferred embodiments, the combination comprises the set E and the set T. The combination of primer sets allows the differential diagnosis between infection and disease caused by SARS-CoV-2 and other Betacoronavirus group B/C such as SARS-CoV and MERS-CoV.

Another aspect of the present invention relates to a kit for the detection of SARS-CoV-2 and/or Betacoronavirus group B/C nucleic acid, comprising at least one pair or set of oligonucleotide primers for amplifying a target sequence of a SARS-CoV-2 nucleic acid according to the present disclosure. Preferably, at least one oligonucleotide of the set or pair comprises a detectable label. The kit optionally comprises reagents for the isothermal amplification of the target sequence and/or the detection of the amplification product. Reagents available for this purpose are well-known in the art and include the DNA polymerases and RT enzymes described above, buffers for the enzymes, detergents, enhancing agents, nucleic acid binding dyes and probes, preferably labelled probes. In some preferred embodiments of the kit of the invention, the primers, and optional reagents are in lyophilised form to allow ambient storage. The components of the kits are packaged together into any of the various containers suitable for nucleic acid amplification such as plates, slides, wells, dishes, beads, particles, cups, strands, chips, strips and others. The kit optionally includes instructions for performing at least one specific embodiment of the method of the invention. In some advantageous embodiments, the kit comprises micro-well plates or microtubes, preferably in a dried format, *i.e.*, wherein the wells of the plates or microtubes comprise a dried composition containing at least the primers, and preferably further comprising all the reagents for the isothermal amplification of the target sequence and/or the detection of the amplification product amplification. In some other advantageous embodiments, the primers and optional reagents are included into any of the devices available for nucleic acid amplification including devices further integrating nucleic acid extraction and/or amplification product detection capacities such as microfluidic devices or other devices. Examples of preferred microfluidic device include the COVIDISC developed by Professor Patrick Tabeling in the Laboratory Chimie Biologie Innovation at ESPCI Paris-PSL.

In some embodiments, the kit is for the diagnosis of SARS-CoV-2 and/or Betacoronavirus group B/C infection or associated diseases such as Covid-19. In some other embodiments, the kit is for the detection of a contamination with SARS-CoV-2 and/or Betacoronavirus group B/C in an environmental sample.

In some preferred embodiments, the kit is for the detection of SARS-CoV-2 and/or Betacoronavirus group B/C by isothermal nucleic acid amplification, comprising at least a set of oligonucleotide primers for isothermal nucleic acid amplification of SARS-CoV-2 nucleic acid or a combination thereof according to the present disclosure. In some particular embodiments, at least one oligonucleotide of the set comprises a detectable label. In some particular embodiments, the kit comprises a first set of oligonucleotide primers which is specific for SARS-CoV-2 and a second set of oligonucleotide primers which is specific for Betacoronavirus group B/C. In some preferred embodiments, the combination comprises the set E and the set T. In some particular embodiments, at least one oligonucleotide of the set comprises a detectable label.

In some embodiments, the kit further comprises at least one oligonucleotide probe or primer for the detection of another agent, in particular another pathogen, for example a human or animal pathogen. In some preferred embodiments, the pathogen is a human pathogen. In some other embodiments, the pathogen is a human pathogen.

Another aspect of the invention relates to the use of a set of oligonucleotide primers, combination or kit according to the present disclosure for the *in vitro* detection of SARS-CoV-2 or Betacoronavirus group B/C nucleic acid in a sample. Betacoronavirus group B/C includes human pathogenic coronaviruses such as SARS-CoV and SARS-CoV-2 (Group B) and MERS-CoV (Group C). Betacoronavirus group B/C are different from other Betacoronavirus groups such as in particular Betacoronavirus group A which include OC43 which can cause the common cold and HKU1.

According to the invention, SARS-CoV-2 may be detected using a set of oligonucleotide primers specific for SARS-CoV-2 or a combination of a set of oligonucleotide primers specific for SARS-CoV-2 and a set of oligonucleotide primers specific for Betacoronavirus group B/C. According to the invention, Betacoronavirus group B/C is detected using a set of oligonucleotide primers specific for Betacoronavirus group B/C. The combination of primer sets allows the differential diagnosis between SARS-CoV-2 infection and disease and infection and disease caused by other Betacoronavirus group B/C such as SARS-CoV and MERS-CoV.

In some embodiments, the set of oligonucleotide primers, combination or kit according to the present disclosure are used for the diagnosis of SARS-CoV-2 and/or Betacoronavirus group B/C infection and associated disease such as Covid-19 in a biological sample from a subject

In some embodiments, the subject is a human. In some other embodiments, the subject is an animal, including companion animals, livestock, and wild or zoo animals. Companion animals includes cat, dog and others.

In some other embodiments, the set of oligonucleotide primers, combination or kit according to the present disclosure is used for the detection of a contamination with SARS-CoV-2 and/or Betacoronavirus group B/C in an environmental sample.

The detection is performed on any sample suspected of containing SARS-CoV-2 and/or Betacoronavirus group B/C. The sample includes any specimen that may contain SARS-CoV-2 or Betacoronavirus group B/C or components thereof such as nucleic acids or fragments of nucleic acids.

Samples include biological samples which refer to any material derived from living or dead individual (human or animal) that may contain SARS-CoV-2 or Betacoronavirus group B/C or target nucleic acid derived therefrom, including any tissue, body fluid or stool. Non-limiting examples of body fluids include whole-blood, serum, plasma, urine, cerebral spinal fluid (CSF), and mucosal secretions, such as with no limitations oral and respiratory tract secretions (sputa, saliva and the like). Samples include swabs such as oral or nasopharyngeal (NP) swabs, aspirate, wash or lavage. Samples for diagnostic tests for SARS-CoV-2 can be taken from the upper (nasopharyngeal/oropharyngeal swabs, nasal aspirate, nasal wash or saliva) or lower respiratory tract (sputum or tracheal aspirate or bronchoalveolar lavage (BAL). In some particular embodiments, the biological sample is a clinical sample (*i.e.*, a sample from living or dead individual (human or animal) suspected of having SARS-CoV-2 or Betacoronavirus group B/C, preferably a body fluid sample, more preferably oral or respiratory tract secretions.

Samples also include environmental samples that may contain SARS-CoV-2 or Betacoronavirus group B/C such as air, water, soil, food, beverages, feed, water (e.g., fresh water, salt water, waste water, and drinking water), sewage, sludge, environmental surfaces and others. The environmental surface sample is for example a surface swab or swipe. Air sample may be collected using an air sampler such as Coriolis Micro (Bertin Instruments). In some particular embodiments, the environmental sample is air or environmental surface.

Samples include direct samples (without nucleic isolation) and processed samples that have been treated to disrupt tissue or cell structure, thus releasing intracellular components into a solution which may further contain reagents (buffers, salts, detergents, enzymes and the like) which are used to prepare, using standard methods, a biological sample for analysis. In particular, processed samples include samples that have been treated by standard methods used for the isolation of nucleic acids from biological samples. In some embodiments, the sample is a direct sample. In some other embodiments, the sample has been treated to isolate nucleic acids.

Another aspect of the invention relates to a method of detection of SARS-CoV-2 and/or Betacoronavirus group B/C nucleic acid in a sample, comprising :
a) subjecting said sample to a nucleic acid amplification reaction using at least one pair or set of oligonucleotide primers for amplifying a target sequence of said viral nucleic acid according to the present disclosure, and
b) detecting the presence or absence of an amplification product for said target sequence.

In step a), of the method of the invention, the oligonucleotide primers which are contacted with the sample suspected of containing a human pathogenic coronavirus (SARS-CoV-2 or Betacoronavirus group B/C) are capable of hybridizing with the target sequence present in any SARS-CoV-2 or Betacoronavirus group B/C target nucleic acid present in said sample. The SARS-CoV-2 or Betacoronavirus group B/C target nucleic acid present in the sample is used as a template for generating an amplification product (amplicon). The amplification product includes monomers and concatemers of the target sequence. The amplification product (amplicon) may be detected by using a DNA binding dye or labelled primer, or hybridization assay using a labelled oligonucleotide probe. In step b) of the method of the invention, the detection of the presence or absence of the amplification product determines the presence or absence of SARS-CoV-2 or Betacoronavirus group B/C virus in the sample, and thereby whether or not the invidual is infected or the environment contaminated with SARS-CoV-2 or Betacoronavirus group B/C.

In some preferred embodiments of the method of the invention, the nucleic acid amplification reaction (step a)) is an isothermal nucleic acid amplification reaction performed with a set of oligonucleotide primers for isothermal nucleic acid amplification according to the present disclosure.

According to the method of the invention, the target sequence is amplified by isothermal nucleic acid amplification reaction using any of the various natural or engineered enzymes available for this purpose. These include in particular DNA polymerases having strong strand- displacement activity in isothermal conditions, thereby obviating the need for thermal cycling. Such polymerases are well-known in the art and include DNA polymerase long fragment (LF) of thermophilic bacteria such as *Bacillus stearothermophilus* (Bst), *Bacillus Smithii* (Bsm), *Geobacillus sp.* M (GspM) and *Thermodesulfatator indicus* (Tin), engineered variants therefrom as well as Taq DNA polymerase variants. Non-limiting examples of polymerase which can be used to perform the method of the invention include Bst LF DNA polymerase, GspM LF DNA polymerase, GspSSD LF DNA polymerase, Tin exo-LF DNA polymerase and SD DNA polymerase which are usually used at 50-75°C, more generally at 55 to 65°C.

In a particular embodiment, the isothermal nucleic acid amplification reaction is performed at 65°C. In another particular embodiment, the isothermal nucleic acid amplification reaction is performed using GspSSD LF DNA polymerase, preferably at 65°C.

According to another particular embodiment of the invention, the isothermal amplification is chosen from strand-displacement amplification (SDA), Rolling-circle amplification (RCA), Cross-priming amplification (CPA), nucleic acid sequence-based amplification (NASBA), Recombinase Polymerase Amplification (RPA) and Loop-mediated amplification (LAMP). The principle of LAMP method is disclosed in Notomi et al., Nucleic Acids Res., 2000 Jun 15; 28(12):e63.

In some embodiments, the amplification product of the isothermal nucleic acid amplification reaction is selected from the group consisting of the sequences SEQ ID NO: 13, 24, 35, 46, 57, 68, 79, 90, 101, 112, 123, 134, 145, 156, 167, 178, 189, 200 and 211; preferably 57 or 189.

Since the viral nucleic acid is RNA, the method of the invention comprise a reverse transcription reaction prior to or concomitantly with the amplification reaction of the target sequence. The reverse transcription reaction is performed using any reverse transcriptase (RT). RT are well-known in the art and include for example Avian Myeloblastosis Virus (AMV) and Moloney Murine Leukemia virus (MMLV) RT. The Reverse transcription and DNA amplification can be performed in the same reaction mixture comprising the DNA polymerase and RT enzymes. In addition, the invention can also use a DNA polymerase having both strong displacement activity and RT activity such as Pyrophage 3173 DNA polymerase.

The amplification product is detected using any of the various methods available for this purpose which are well-known in the art. For example, the detection method is fluorescence, bioluminescence, colorimetry, turbidimetry, immunoenzymatic or electrochemical detection. The detection may be semi-quantitative or quantitative. The detection may also be real-time detection, wherein the signal resulting from the presence of the amplification product is measured during the course of the nucleic acid amplification reaction to monitor the accumulation of specific amplification products. Fluorescence may use DNA intercaling dyes, fluorescent molecular beacon probes or a fluorescence metal indicator such as calcein. Colorimetry may use a colored indicator for alkaline metal ions, such as hydroxy naphthol blue (Goto et al., BioTechniques. 2009 Mar;46(3): 167-72) or pH indicators (Tanner et al., BioTechniques, 2015 Feb;58(2):59-68). Turbidity is used when the amplification reaction, such as LAMP, produces large amounts of magnesium pyrophosphate (a white precipitate) and dsDNA, which allow visual inspection of results using a turbidimeter (Mori et al., Biochem. Biophys. Res. Commun., 2001 Nov 23; 289(1): 150-4). Electrochemical detection may use a pH meter for direct measurement of released hydrogen ions during the amplification reaction, such as LAMP (Xie S et al., Chem. Commun., 2014 Oct 24; 50(100):15932-5), or integrated electrodes for measuring decreases in current resulting from increasing binding of electrochemically-active DNA-binding redox reporters, such as Methylene Blue, to amplification products (Xie et al., Biosens. Bioelectron. 2014 May 15; 55:324-9). Immunoassays include Enzyme-linked immunosorbent assays (ELISA) and lateral flow immunoassays based on the use of specific probes (Tsai S-M et al., J. Virol. Methods. 2012 Apr; 181(1):117-24; Ravan H and Yazdanparast R., Anal. Biochem., 2013 Aug 15; 439(2): 102-8). Bioluminescence detection may be through measurement of bioluminescent output of the coupled conversion of inorganic pyrophosphate produced stoichiometrically during nucleic acid synthesis to ATP by the enzyme ATP sulfurylase (Gandelman et al., PLoS ONE., 2010 Nov 30; 5(11)).

According to another preferred embodiment, the method comprises at least one non-SARS-CoV-2 internal control (IC) nucleic acid that is processed as the tested samples and amplified in the same assay reaction mixtures by using amplification oligonucleotide primers specific for a target sequence of the IC nucleic acid sequence. In some embodiments, the IC target sequence is selected from the group consisting of: the sequence from positions 26 to 293, 227 to 498, or 387 to 621 of the CDS of human GAPDH gene (positions 189 to 1196 of GenBank NM_002046.5.2) and the sequence from positions 204 to 517, 584 to 869, or 908 to 1148 of the CDS of *Drosophila melanogaster* Sigma virus G gene (positions 4262 to 5947 of GenBank GQ375258).

According to another preferred embodiment, the method comprises assaying several samples simultaneously. In some advantageous embodiments, the method comprises assaying multiple samples simultaneously in a high-throughput process.

The method of the invention is useful for the diagnosis of SARS-CoV-2 and associated disease (Covid-19) in a biological sample from a subject. The method is also useful for the differential diagnosis between SARS-CoV-2 disease (Covid-19) and other Betacoronavirus group B/C diseases such as SARS-CoV and MERS-CoV diseases. The method is also useful for the detection of an environmental contamination with SARS-CoV-2. The method is also useful for the differential detection between a contamination with SARS-CoV-2 and a contamination with other Betacoronavirus group B/C such as SARS-CoV and MERS-CoV. In some embodiments, the subject is a human subject. In other embodiments, the subject is an animal including companion animals, livestock, and wild or zoo animals. Companion animals include cat, dog and others.

In some embodiments, the method is a method of decontamination of an environment suspected to be contaminated with SARS-CoV-2 or Betacoronavirus group B/C, comprising the steps of :
a) subjecting an environmental sample suspected to be contaminated with SARS-CoV-2 or Betacoronavirus group B/C to a nucleic acid amplification reaction using at least one pair or set of oligonucleotide primers for amplifying a target sequence of said viral nucleic acid according to the present disclosure;
b) detecting the presence or absence of an amplification product for said target sequence; and
c) decontaminating the environment tested positive in step (b).

The decontamination may be performed by standard method that are well-known in the art using standard disinfectants that kill viruses including SARS-CoV-2 and/or Betacoronavirus group B/C.

In some embodiments, the method is a method of controlling an infection with SARS-CoV-2 or Betacoronavirus group B/C, comprising the steps of:
a) subjecting biological sample(s) from subject(s) suspected to be contaminated with SARS-CoV-2 or Betacoronavirus group B/C to a nucleic acid amplification reaction using at least one pair or set of oligonucleotide primers for amplifying a target sequence of said viral nucleic acid according to the present disclosure;
b) detecting the presence or absence of an amplification product for said target sequence; and
c) isolating and/or treating subject(s) tested positive in step (b).

The treatment in step (c) may be a symptomatic and/or antiviral treatment using standard medicaments that are well-known in the art.

In some embodiments, the method comprises repeating the nucleic acid detection according to steps (a) and (b) at least one time after the initiation of the treatment and/or isolation and continuing the isolation and/or treatment if the patient is tested positive or stopping the isolation and/or treatment if the patient is tested negative.

Another aspect of the invention is an oligonucleotide primer of the set of oligonucleotide primers according to the present disclosure. The oligonucleotide primer, in particular a labeled oligonucleotide primer is useful as a probe for the detection of SARS-CoV-2 nucleic acid including the specific detection of SARS-CoV-2 or Betacoronavirus group B/C nucleic acid.

The practice of the present invention will employ, unless otherwise indicated, conventional techniques, which are within the skill of the art. Such techniques are explained fully in the literature.

The invention will now be exemplified with the following examples, which are not limitative, with reference to the attached drawings in which:

### FIGURE LEGENDS

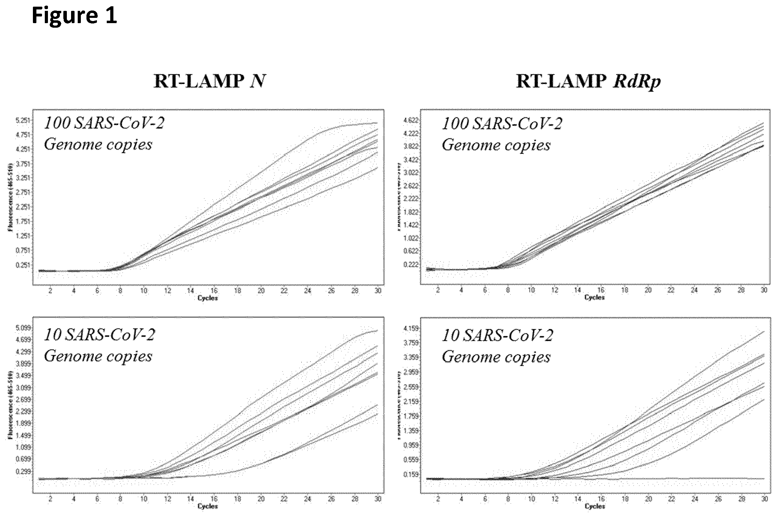

### Figure 1. Amplification curves obtained for 10 and 100 genome copies of SARS-CoV-2 RNA template in a real-time SARS-CoV-2 RT-LAMP N and RdRp assay

### EXAMPLES

### 1. Material and methods

Specific target gene regions were selected by multiple sequence alignments of all genome sequences available in the GISAID database for SARS-CoV-2 (as of March 27) and primers specifically targeting selected gene regions were designed. Primer specificity was verified *in silico* by BLAST analysis. A total of 40 different primer set, each composed of 6 specific primers, were designed: 11 primer sets targeting the nucleoprotein (*N*) gene; 11 primer sets targeting the RNA-dependent RNA polymerase (*RdRp*) gene; and 7 primer sets targeting the *ORF3a*/*ORF4* region and 11 primer sets targeting the *ORF4*/*ORF5* region. In addition, two published RT-LAMP primer sets targeting the *ORF1a*/*b* were included in the performance evaluation study for comparison (Lamb et al., PLOS ONE, 2020, June 12, 15:e0234682, doi: 10.1371; Yu et al., Clin. Chem., 2020 April 21, hvaa102.doi: 10.1093).

RNA is extracted from specimens using the CE-IVD NucleoSpin^{®} Dx Virus kit (Macherey Nagel ref. 740895.50) or BEC SARS-CoV-2 RT-LAMP kit for human samples, (ref. n° P05301).

A generic real-time RT-LAMP protocol was developed for real-time detection of target SARS-CoV-2 genes. Briefly, a 25 µL RT-LAMP reaction was set up containing 15µL of ISO-004 master mix (Optigene), 1,3µL of the external, internal and loop primer mixture (final concentrations of 200, 1600 and 800 nM, respectively) and 5 µL template RNA. Primer sets were compared individually using the generic RT-LAMP protocol for optimal amplification and reaction time. Amplification was performed at a constant temperature of 65°C for 30 minutes. All reactions were conducted in a LightCycler 480 (Roche) or an ISO-8 (Axxin) instrument.

Controls included a positive control (internal quality control) consisting of an RNA extract, obtained from a pool of SARS-CoV-2 Coronavirus positive samples, diluted to a concentration of 20 genomic copies per µL (equivalent to a cycle threshold (Ct) of 35 in real-time RT-PCR.

To determine the analytical sensitivity of the *SARS-CoV-2* RT-LAMP assays, serial 10-fold dilutions of genomic *SARS-CoV-2* (extracted from a pool of CIBU107 and CIBU132 strains) were tested in parallel with the reference real-time RT-PCR developed by the National Reference Centre (NRC) for Respiratory Viruses in France (WHO Coronavirus disease COVID-19 technical guidance: Laboratory testing for 2019-nCoV in humans, available from https://www.who.int/docs/default-source/coronaviruse/whoinhouseassays.pdf? sfvrsn=de3a76aa_2) and the *SARS-CoV-2* RT-LAMP assays. Each dilution of template RNA was tested 8 times to determine intra-assay variability.

The specificity and sensitivity of the *SARS-CoV-2* RT-LAMP assays was determined on 45 respiratory specimen extracts from suspect cases diagnosed negative for SARS-CoV-2 and 62 respiratory specimen extracts from suspect cases diagnosed positive for SARS-CoV-2 were tested in the RT-LAMP assay.

### 2. Results

The *RdRp* gene encoding the RNA-dependent RNA polymerase and the *N* gene encoding the nucleoprotein were selected as optimal targets for the detection of SARS-CoV-2 virus. The RT-LAMP assays primer list is presented in **Table 1.**

Serial 10-fold dilutions of genomic SARS-CoV-2 (extracted from a pool of CIBU107 and CIBU132 strains) were prepared, aliquoted and tested 8 times using the various RT-LAMP master mixes to determine conditions for optimal reaction efficiency.

**Table 2: Optimization of RT-LAMP assay reaction master mix compositions**

| **RT-LAMP Primer Set ID** | **Limit of detection (genome copies per reaction)** | **Mean time to result (min)** |
|---|---|---|
| **A*** | 10 | 25,41 |
| **B** | 10 | 24,26 |
| **C** | 100 | 7,45 |
| **D** | 100 | 23,31 |
| **E** | **10** | **11,48** |
| **F** | 10 | 9,91 |
| **G** | 100 | 9,2 |
| **H** | 100 | 14,28 |
| **I** | 100 | 13,59 |
| **J** | 100 | 21,35 |
| **K** | 100 | 19,9 |
| **L**** | 10 | 16,26 |
| **M** | 10 | 17,14 |
| **N** | 100 | 10,14 |
| **O** | 100 | 11,63 |
| **P** | 100 | 11,45 |
| **T** | **10** | **11,21** |
| **U** | 10 | 15,22 |
| **V** | 10 | 16,48 |
| **AC***** | 1000 | 25,00 |
| **Yu et al. 2020°** | 100 | 11,67 |
| **Lamb et al. 2020°** | 10 | 11,9 |

| | | |
|---|---|---|
| * N gene; ** RdRp; *** ORF3a/ORF4 °ORF1a/b | | |

**Table 3. SARS-CoV-2 RT-LAMP assay primer list**

| Target gene | Primer ID | Primer type | Primer sequence |
|---|---|---|---|
| *N* gene | 116 | External | TCAAGGAACAACATTGCCAA (SEQ ID NO: 47) |
| (set E) | 74 | External | CTTCTTAGAAGCCTCAGCAG (SEQ ID NO: 48) |
| | 193 | Internal | GCCAGCCATTCTAGCAGGAGGTAGTCGCAACAGTTC AAGA (SEQ ID NO : 49) |
| | 160 | Internal | AATGGCGGTGATGCTGCTTCTCAAGCTGGTTCAATC TG (SEQ ID NO : 50) |
| | 130 | Loop | TGCTGCCTGGAGTTGAATT (SEQ ID NO: 51) |
| | 150 | Loop | TTGCTTTGCTGCTGCTTG (SEQ ID NO: 52) |
| *RdRP* gene | 56 | External | CCTTATGGGTTGGGATTATCC (SEQ ID NO: 179) |
| (set T) | 140 | External | TGTGGCATCTCCTGATGA (SEQ ID NO: 180) |
| | 177 | Internal | CACGTTGTATGTTTGCGAGCAAGTGATAGAGCCATG CCTAAC (SEQ ID NO : 181) |
| | 205 | Internal | TGTAGCTTGTCACACCGTTTCTCCACACATGACCATT TCACT ((SEQ ID NO : 182) |
| | 28 | Loop | AGTGAGGCCATAATTCTAAGCA (SEQ ID NO: 183) |
| | 24 | Loop | AGCTAATGAGTGTGCTCAAGT (SEQ ID NO: 184) |

Among the tested RT-LAMP primer sets, primer sets E and T were identified as best performing primers as they demonstrated the lowest limit of detection with the fastest time-to-result, and were not associated with non-specific amplification due to self-priming artifacts. (**Tables 2 & 3**).

Serial dilutions of *SARS-CoV-2* genomic RNA were used to determine the analytical sensitivity of the RT-LAMP assays. The *SARS-CoV-2* RT-LAMP assay demonstrated a limit of detection of 10 genome copies per reaction, with a time-to-result of less than 15 minutes (**Table 4**).

**Table 4. Analytical sensitivity (limit-of-detection) of the RT-LAMP assays as compared to the gold-standard RT-PCR assay**

| Assay Target gene | | Primer set ID | Quantified genomic SARS-CoV-2 RNA | | | | | |
|---|---|---|---|---|---|---|---|---|
| | | | 10 genome copies | | | 100 genome copies | | |
| | | | Mean Cp | SD Cp | Detected replicates (out of 8) | Mean Cp | SD Cp | Detected replicates (out of 8) |
| **Real-time RT-PCR** | | | | | | | | |
| | *RdRp* | IP2 (FAM) | 37.02 | 0.56 | 8 | 34.65 | 0.48 | 8 |
| | | IP4 (HEX) | 38.18 | 0.86 | 7 | 35.05 | 0.36 | 8 |

| **Real-time RT-LAMP** | | | | | | | | |
|---|---|---|---|---|---|---|---|---|
| | *N* | **E** | **11.48** | **2.66** | **8** | **7.95** | **0.38** | **8** |
| | *RdRp* | **T** | **11.21** | **2.47** | **7** | **7.59** | **0.71** | **8** |
| | *ORFlab* | Yu *et* al., 2020 | 14.34 | - | 1 | **11.68** | 0.95 | 8 |
| | *ORFlab* | Lamb *et* al., 2020 | 11.90 | 1.51 | 8 | 8.57 | 0.48 | 8 |

Amplification curves obtained by RT-LAMP for 10 and 100 genome copies of *SARS-CoV-2* RNA per reaction are shown in **Figure 1****.**

The recognition specificity of real-time RT-LAMP primers selected for the detection of Betacoronaviruses and SARS-CoV-2 was determined in silico based on the analysis of sequences available in international databases. It was also experimentally controlled on extracts of human pathogenic RNA viruses commonly handled in the laboratory:
- Betacoronavirus group B/C: MERS-Coronavirus and SARS-Coronavirus
- Coronavirus OC43 (Betacoronavirus group A)
- Coronavirus 229E (alphacoronavirus)
- Influenza A/H1N1
- Influenza A/H5N1
- Influenza A/H3N2
- Influenza B

Specificity was also evaluated on 45 respiratory specimen extracts from suspect cases diagnosed negative for SARS-CoV-2.

No cross-detection was found between the methods evaluated and the DNA/RNA viruses commonly handled in the laboratory. Therefore, no false-positive results were observed for the different tests. The specificity is above the laboratory's threshold for rapid tests (98%), **(Table 5)**.

The sensitivity of the tests was evaluated on 62 respiratory specimen extracts from suspect cases diagnosed positive for SARS-CoV-2. The sensitivity is above the threshold retained by the laboratory for rapid tests (95%), **(Table 5).**

**Table 5: Clinical sensitivity and specificity**

| | | |
|---|---|---|
| **RT-LAMP assay** | **Results** | **Analytic Performances** |
| βCoV group B/C | True positives 62/64 | |
| | False positives 0/51 | Specificity = 100 % |
| | True negatives 51/51 | Sensitivity = 97 % |
| | False negatives 2/64 | |
| SARS-CoV-2 | True positives 59/62 | |
| | False positives 0/53 | Specificity = 100 % |
| | True negatives 53/53 | Sensitivity = 95 % |
| | False negatives 3/62 | |

A subset of 10 patient swab extracts, collected from 4 SARS-CoV-2 cases and 6 negative SARS-CoV-2 individuals were tested by RT-LAMP using primer subsets E, T and the primer set published by Lamb et al. While all primer sets were equally specific (no false positive results), primer sets E and T demonstrated superior clinical sensitivity (100%) as compared to the primer set from Lamb et al on the tested SARS-CoV-2 positive samples (50%) (**Table 6**).

**Table 6: Comparison of RT-LAMP assay performances on a panel of clinical samples**

| RT-LAMP Primer set ID | Number of positive samples detected | Number of negative samples detected |
|---|---|---|
| E | 4/4 | 6/6 |
| T | 4/4 | 6/6 |
| Lamb et al., 2020 | 2/4 | 6/6 |

In conclusion, the developed RT-LAMP method demonstrated equivalent analytical sensitivity and a faster time-to-result (30 minutes as compared to 1 hour 45 minutes) as compared to the currently used reference real-time RT-PCR assays, making it highly suitable for rapid, reliable diagnostic applications. In addition the selected optimized RT-LAMP primer sets (E and T) showed either increased amplification speed or a lower limit-of-detection as compared to published primer sets. Moreover, the selected optimized RT-LAMP primer sets (E and T) demonstrated superior clinical sensitivity (100%) as compared to published primer set (50 %).

| **Table 1. RT-LAMP assays primer list** | | | | | | | | |
|---|---|---|---|---|---|---|---|---|
| **Primer Set ID** 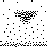 | **Target genome (accession nr)** 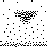 | **Target region** 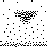 | **Position** 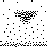 | **Name** 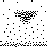 | **Sequence (5' to 3')** 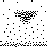 | **Primer position** 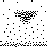 | **Primer length** 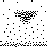 | **SEQ ID No:** |
| **A** | SARS-CoV-2 | N gene | 450 | F3 | TCCTGCTAACAATGCTGC | 450 | 18 | 3 |
| | (NC_045512) | | 694 | B3 | TCTCAAGCTGGTTCAATCTG | 694 | 20 | 4 |
| | | | | FIP(F1c+F2) | AACGAGAAGAGGCTTGACTGCTCAAGGAACAACATTGCCA | | 40 | 5 |
| | | | | BIP(B1c+B2) | CTCATCACGTAGTCGCAACAGTATTGCCAGCCATTCTAGC | | 40 | 6 |
| | | | 524 | LoopF | CCTTCTGCGTAGAAGCCTT | 524 | 19 | 7 |
| | | | 586 | LoopB | AATTCAACTCCAGGCAGCA | 586 | 19 | 8 |
| | | | 486 | F2 | TCAAGGAACAACATTGCCA | 486 | 19 | 9 |
| | | | 556 | F1c | AACGAGAAGAGGCTTGACTGC | 556 | 21 | 10 |
| | | | 639 | B2 | ATTGCCAGCCATTCTAGC | 639 | 18 | 11 |
| | | | 558 | B1c | CTCATCACGTAGTCGCAACAGT | 558 | 22 | 12 |
| | | | | Product | TCAAGGAACAACATTGCCAAAAGGCTTCTACGCAGAAGGGAGCAGAG GCGGCAGTCAAGCCTCTTCTCGTTCCTCATCACGTAGTCGCAACAGTT CAAGAAATTCAACTCCAGGCAGCAGTAGGGGAACTTCTCCTGCTAGAA TGGCTGGCAAT | | 154 | 13 |
| **B** | SARS-CoV-2 | N gene | 486 | F3 | TCAAGGAACAACATTGCCA | 486 | 19 | 14 |
| | (NC_045512) | | 771 | B3 | CTTCTTAGAAGCCTCAGCAG | 771 | 20 | 15 |
| | | | | F\|P(F1c+F2) | GCCAGCCATTCTAGCAGGAGCACGTAGRCGCAACAGTT | | 38 | 16 |
| | | | | BIP(B1c+B2) | AATGGCGGTGATGCTGCTTCTCAAGCTGGTTCAATCTG | | 38 | 17 |
| | | | 604 | LoopF | TGCTGCCTGGAGTTGAATT | 604 | 19 | 18 |
| | | | 656 | LoopB | TTGCTTTGCTGCTGCTTG | 656 | 18 | 19 |
| | | | 563 | F2 | CACGTAGTCGCAACAGTT | 563 | 18 | 20 |
| | | | 636 | F1c | GCCAGCCATTCTAGCAGGAG | 636 | 20 | 21 |
| | | | 694 | B2 | TCTCAAGCTGGTTCAATCTG | 694 | 20 | 22 |
| | | | 637 | B1c | AATGGCGGTGATGCTGCT | 637 | 18 | 23 |
| | | | | Product | CACGTAGTCGCAACAGTTCAAGAAATTCAACTCCAGGCAGCAGTAGG GGAACTTCTCCTGCTAGAATGGCTGGCAATGGCGGTGATGCTGCTCTT GCTTTGCTGCTGCTTGACAGATTGAACCAGCTTGAGA | | 132 | 24 |

| **Primer Set ID** | **Target genome (accession nr)** | **Target region** | **Position** | **Name** | **Sequence (5' to 3')** | **Primer position** | **Primer length** | **SEQ ID NO:** |
|---|---|---|---|---|---|---|---|---|
| **C** | SARS-CoV-2 | N gene | 486 | F3 | TCAAGGAACAACATTGCCA | 486 | 19 | 25 |
| | (NC_045512) | | 771 | B3 | CTTCTTAGAAGCCTCAGCAG | 771 | 20 | 26 |
| | | | | RP(F1c+F2) | GCCAGCCATTCTAGCAGGAGCGTAGTCGCAACAGTTCA | | 38 | 27 |
| | | | | BIP(B1c+B2) | AATGGCGGTGATGCTGCTTCTCAAGCTGGTTCAATCTG | | 38 | 28 |
| | | | 604 | LoopF | TGCTGCCTGGAGTTGAATT | 604 | 19 | 29 |
| | | | 656 | LoopB | TTGCTTTGCTGCTGCTTG | 656 | 18 | 30 |
| | | | 565 | F2 | CGTAGTCGCAACAGTTCA | 565 | 18 | 31 |
| | | | 636 | F1c | GCCAGCCATTCTAGCAGGAG | 636 | 20 | 32 |
| | | | 694 | B2 | TCTCAAGCTGGTTCAATCTG | 694 | 20 | 33 |
| | | | 637 | B1c | AATGGCGGTGATGCTGCT | 637 | 18 | 34 |
| | | | | Product | CGTAGTCGCAACAGTTCAAGAAATTCAACTCCAGGCAGCAGTAGGGG AACTTCTCCTGCTAGAATGGCTGGCAATGGCGGTGATGCTGCTCTTGC TTTGCTGCTGCTTGACAGATTGAACCAGCTTGAGA | | 130 | 35 |
| **D** | SARS-CoV-2 | N gene | 468 | F3 | AATCGTGCTACAACTTCCTC | 468 | 20 | 36 |
| | (NC_045512) | | 759 | B3 | CTCAGCAGCAGATTTCTTAGT | 759 | 21 | 37 |
| | | | | FIP(F1c+F2) | CCTACTGCTGCCTGGAGTTGAGTCAAGCCTCTTCTCGT | | 38 | 38 |
| | | | | BIP(B1c+B2) | CTCCTGCTAGAATGGCTGGCTCAATCTGTCAAGCAGCAG | | 39 | 39 |
| | | | 575 | LoopF | TTGCGACTACGTGATGAGG | 575 | 19 | 40 |
| | | | 643 | LoopB | GGTGATGCTGCTCTTGCT | 643 | 18 | 41 |
| | | | 538 | F2 | AGTCAAGCCTCTTCTCGT | 538 | 18 | 42 |
| | | | 609 | F1c | CCTACTGCTGCCTGGAGTTG | 609 | 20 | 43 |
| | | | 682 | B2 | TCAATCTGTCAAGCAGCAG | 682 | 19 | 44 |
| | | | 617 | B1c | CTCCTGCTAGAATGGCTGGC | 617 | 20 | 45 |
| | | | | Product | AGTCAAGCCTCTTCTCGTTCCTCATCACGTAGTCGCAACAGTTCAAGA AATTCAACTCCAGGCAGCAGTAGGGGAACTTCTCCTGCTAGAATGGCT GGCAATGGCGGTGATGCTGCTCTTGCTTTGCTGCTGCTTGACAGATTG A | | 145 | 46 |
| **E** | SARS-CoV-2 | N gene | 486 | F3 | TCAAGGAACAACATTGCCAA | 486 | 20 | 47 |
| | (NC_045512) | | 771 | B3 | CTTCTTAGAAGCCTCAGCAG | 771 | 20 | 48 |
| | | | | FIP(F1cfF2) | GCCAGCCATTCTAGCAGGAGGTAGTCGCAACAGTTCAAGA | | 40 | 49 |
| | | | | BIP(B1c+B2) | AATGGCGGTGATGCTGCTTCTCAAGCTGGTTCAATCTG | | 38 | 50 |
| | | | 604 | LoopF | TGCTGCCTGGAGTTGAATT | 604 | 19 | 51 |
| | | | 656 | LoopB | TTGCTTTGCTGCTGCTTG | 656 | 18 | 52 |
| | | | 566 | F2 | GTAGTCGCAACAGTTCAAGA | 566 | 20 | 53 |
| | | | 636 | F1c | GCCAGCCATTCTAGCAGGAG | 636 | 20 | 54 |
| | | | 694 | B2 | TCTCAAGCTGGTTCAATCTG | 694 | 20 | 55 |
| | | | 637 | B1c | AATGGCGGTGATGCTGCT | 637 | 18 | 56 |
| | | | | Product | GTAGTCGCAACAGTTCAAGAAATTCAACTCCAGGCAGCAGTAGGGGA ACTTCTCCTGCTAGAATGGCTGGCAATGGCGGTGATGCTGCTCTTGCT TTGCTGCTGCTTGACAGATTGAACCAGCTTGAGA | | 129 | 57 |
| **F** | SARS-CoV-2 | N gene | 538 | F3 | AGTCAAGCCTCTTCTCGT | 538 | 18 | 58 |
| | (NC_045512) | | 779 | B3 | TGCCGAGGCTTCTTAGAA | 779 | 18 | 59 |
| | | | | FIP(F1c+F2) | AGCAGCATCACCGCCATTAATTCAACTCCAGGCAGC | | 36 | 60 |
| | | | | BIP(B1c+B2) | GCTTTGCTGCTGCTTGACAGTTGTTGGCCTTTACCAGAC | | 39 | 61 |
| | | | 635 | LoopF | CCAGCCATTCTAGCAGGAG | 635 | 19 | 62 |
| | | | 678 | LoopB | ATTGAACCAGCTTGAGAGCA | 678 | 20 | 63 |
| | | | 586 | F2 | AATTCAACTCCAGGCAGC | 586 | 18 | 64 |
| | | | 654 | F1c | AGCAGCATCACCGCCATT | 654 | 18 | 65 |
| | | | 720 | B2 | TTGTTGGCCTTTACCAGAC | 720 | 19 | 66 |
| | | | 658 | B1c | GCTTTGCTGCTGCTTGACAG | 658 | 20 | 67 |
| | | | | Product | AATTCAACTCCAGGCAGCAGTAGGGGAACTTCTCCTGCTAGAATGGCT GGCAATGGCGGTGATGCTGCTCTTGCTTTGCTGCTGCTTGACAGATTG AACCAGCTTGAGAGCAAAATGTCTGGTAAAGGCCAACAA | | 135 | 68 |
| **G** | SARS-CoV-2 | N gene | 73 | F3 | GGCAGTAACCAGAATGGAG | 73 | 19 | 69 |
| | (NC_045512) | | 328 | B3 | AATACCATCTTGGACTGAGATC | 328 | 22 | 70 |
| | | | | FIP(F1c+F2) | TTGGAACGCCTTGTCCTCGATACTGCGTCTTGGTTCAC | | 38 | 71 |
| | | | | BIP(B1c+B2) | ACCAATAGCAGTCCAGATGACCCGTCACCACCACGAATTC | | 40 | 72 |
| | | | 186 | LoopF | TTCCTTGCCATGTTGAGTGA | 186 | 20 | 73 |
| | | | 249 | LoopB | AATTGGCTACTACCGAAGAGC | 249 | 21 | 74 |
| | | | 143 | F2 | ATACTGCGTCTTGGTTCAC | 143 | 19 | 75 |
| | | | 220 | F1c | TTGGAACGCCTTGTCCTCG | 220 | 19 | 76 |
| | | | 295 | B2 | CGTCACCACCACGAATTC | 295 | 18 | 77 |
| | | | 226 | B1c | ACCAATAGCAGTCCAGATGACC | 226 | 22 | 78 |
| | | | | Product | ATACTGCGTCTTGGTTCACCGCTCTCACTCAACATGGCAAGGAAGACC TTAAATTCCCTCGAGGACAAGGCGTTCCAATTAACACCAATAGCAGTC CAGATGACCAAATTGGCTACTACCGAAGAGCTACCAGACGAATTCGTG GTGGTGACG | | 153 | 79 |
| **H** | SARS-CoV-2 | N gene | 208 | F3 | CAAGGCGTTCCAATTAACAC | 208 | 20 | 80 |
| | (NC_045512) | | 467 | B3 | GCAGCATTGTTAGCAGGA | 467 | 18 | 81 |
| | | | | FIP(F1c+F2) | ACCGTCACCACCACGAATTCCAATAGCAGTCCAGATGACC | | 40 | 82 |
| | | | | BIP(B1c+B2) | CTAGGAACTGGGCCAGAAGCACCCATATGATGCCGTCT | | 38 | 83 |
| | | | 269 | LoopF | GCTCTTCGGTAGTAGCCAATT | 269 | 21 | 84 |
| | | | 359 | LoopB | GACTTCCCTATGGTGCTAACAA | 359 | 22 | 85 |
| | | | 228 | F2 | CAATAGCAGTCCAGATGACC | 228 | 20 | 86 |
| | | | 297 | F1c | ACCGTCACCACCACGAATTC | 297 | 20 | 87 |
| | | | 398 | B2 | ACCCATATGATGCCGTCT | 398 | 18 | 88 |
| | | | 337 | B1c | CTAGGAACTGGGCCAGAAGC | 337 | 20 | 89 |
| | | | | Product | CAATAGCAGTCCAGATGACCAAATTGGCTACTACCGAAGAGCTACCAG ACGAATTCGTGGTGGTGACGGTAAAATGAAAGATCTCAGTCCAAGATG GTATTTCTACTACCTAGGAACTGGGCCAGAAGCTGGACTTCCCTATGG TGCTAACAAAGACGGCATCATATGGGT | | 171 | 90 |
| **I** | SARS-CoV-2 | N gene | 171 | F3 | TCAACATGGCAAGGAAGAC | 171 | 19 | 91 |
| | (NC_045512) | | 467 | B3 | GCAGCATTGTTAGCAGGA | 467 | 18 | 92 |
| | | | | FIP(F1c+F2) | ACCGTCACCACCACGAATTCTTAACACCAATAGCAGTCCAG | | 41 | 93 |
| | | | | BIP(B1c+B2) | CTAGGAACTGGGCCAGAAGCACCCATATGATGCCGTCT | | 38 | 94 |
| | | | 269 | LoopF | GCTCTTCGGTAGTIGCCAATT | 269 | 21 | 95 |
| | | | 359 | LoopB | GACTTCCCTATGGTGCTAACAA | 359 | 22 | 96 |
| | | | 221 | F2 | TTAACACCAATAGCAGTCCAG | 221 | 21 | 97 |
| | | | 297 | F1c | ACCGTCACCACCACGAATTC | 297 | 20 | 98 |
| | | | 398 | B2 | ACCCATATGATGCCGTCT | 398 | 18 | 99 |
| | | | 337 | B1c | CTAGGAACTGGGCCAGAAGC | 337 | 20 | 100 |
| | | | | Product | TTAACACCAATAGCAGTCCAGATGACCAAATTGGCTACTACCGAAGAG CTACCAGACGAATTCGTGGTGGTGACGGTAAAATGAAAGATCTCAGTC CAAGATGGTATTTCTACTACCTAGGAACTGGGCCAGAAGCTGGACTTC CCTATGGTGCTAACAAAGAC GGCATCATATGGGT | | 178 | 101 |
| **J** | SARS-CoV-2 | N gene | 171 | F3 | TCAACATGGCAAGGAAGAC | 171 | 19 | 102 |
| | (NC_045512) | | 467 | B3 | GCAGCATTGTTAGCAGGA | 467 | 18 | 103 |
| | | | | FIP(F1c+F2) | ACCGTCACCACCACGAATTCCCAATAGCAGTCCAGATGAC | | 40 | 104 |
| | | | | BIP(B1c+B2) | CTAGGAACTGGGCCAGAAGCACCCATATGATGCCGTCT | | 38 | 105 |
| | | | 269 | LoopF | GCTCTTCGGTAGTAGCCAATT | 269 | 21 | 106 |
| | | | 359 | LoopB | GACTTCCCTATGGTGCTAACAA | 359 | 22 | 107 |
| | | | 227 | F2 | CCAATAGCAGTCCAGATGAC | 227 | 20 | 108 |
| | | | 297 | F1c | ACCGTCACCACCACGAATTC | 297 | 20 | 109 |
| | | | 398 | B2 | ACCCATATGATGCCGTCT | 398 | 18 | 110 |
| | | | 337 | B1c | CTAGGAACTGGGCCAGAAGC | 337 | 20 | 111 |
| | | | | Product | CCAATAGCAGTCCAGATGACCAAATTGGCTACTACCGAAGAGCTACCA GACGAATTCGTGGTGGTGACGGTAAAATGAAAGATCTCAGTCCAAGAT GGTATTTCTACTACCTAGGAACTGGGCCAGAAGCTGGACTTCCCTATG GTGCTAACAAAGACGGCATCATATGGGT | | 172 | 112 |
| **K** | SARS-CoV-2 | N gene | 208 | F3 | CAAGGCGTTCCAATTAACAC | 208 | 20 | 113 |
| | (NC_045512) | | 467 | B3 | GCAGCATTGTTAGCAGGA | 467 | 18 | 114 |
| | | | | FIP(F1c+F2) | ACCGTCACCACCACGAATTCATAGCAGTCCAGATGACCA | | 39 | 115 |
| | | | | BIP(B1c+B2) | CTAGGAACTGGGCCAGAAGCACCCATATGATGCCGTCT | | 38 | 116 |
| | | | 269 | LoopF | GCTCTTCGGTAGTAGCCAATT | 269 | 21 | 117 |
| | | | 359 | LoopB | GACTTCCCTATGGTGCTAACAA | 359 | 22 | 118 |
| | | | 230 | F2 | ATAGCAGTCCAGATGACCA | 230 | 19 | 119 |
| | | | 297 | F1c | ACCGTCACCACCACGAATTC | 297 | 20 | 120 |
| | | | 398 | B2 | ACCCATATGATGCCGTCT | 398 | 18 | 121 |
| | | | 337 | B1c | CTAGGAACTGGGCCAGAAGC | 337 | 20 | 122 |
| | | | | Product | ATAGCAGTCCAGATGACCAAATTGGCTACTACCGAAGAGCTACCAGAC GAATTCGTGGTGGTGACGGTAAAATGAAAGATCTCAGTCCAAGATGGT ATTTCTACTACCTAGGAACTGGCCCAGAAGCTGGACTTCCCTATGGTG CTAACAAAGACGGCATCATATGGGT | | 169 | 123 |
| **L** | SARS-CoV-2 | Rd Rp gene | 1 782 | F3 | TATGGTGGTTGGCACAAC | 1782 | 18 | 124 |
| | (NC_045512) | | 2 057 | B3 | TGTGGCATCTCCTGATGA | 2 057 | 18 | 125 |
| | | | | FIP(F1c+F2) | TGCGAGCAAGAACAAGTGAGGTGGGTTGGGATTATCCTAAATG | | 43 | 126 |
| | | | | BIP(B1c+B2) | TGTTGTAGCTTGTCACACCGTTCCACACATGACCATTTCACT | | 42 | 127 |
| | | | 1 890 | LoopF | TAAGCATGTTAGGCATGGCT | 1890 | 20 | 128 |
| | | | 1 964 | LoopB | AGCTAATGAGTGTGCTCAAGT | 1964 | 21 | 129 |
| | | | 1 843 | F2 | TGGGTTGGGATTATCCTAAATG | 1843 | 22 | 130 |
| | | | 1920 | F1c | TGCGAGCAAGAPCAAGTGAGG | 1920 | 21 | 131 |
| | | | 2 008 | B2 | CCACACATGACCATTTCACT | 2 008 | 20 | 132 |
| | | | 1 932 | B1c | TGTTGTAGCTTGTCACACCGTT | 1932 | 22 | 133 |
| | | | | Product | TGGGTTGGGATTATCCTAAATGTGATAGAGCCATGCCTAACATGCTTA GAATTATGGCCTCACTTGTTCTTGCTCGCAAACATACAACGTGTTGTAG CTTGTCACACCGTTTCTATAGATTAGCTAATGAGTGTGCTCAAGTATTG AGTGAAATGGTCATGTGTGG | | 166 | 134 |
| **M** | SARS-CoV-2 | RdRp gene | 1 782 | F3 | TATGGTGGTTGGCACAAC | 1782 | 18 | 135 |
| | (NC_045512) | | 2 057 | B3 | TGTGGCATCTCCTGATGA | 2 057 | 18 | 136 |
| | | | | FIP(F1c+F2) | TGCGAGCAAGAACAAGTGAGGGGGTTGGGATTATCCTAAATGT | | 43 | 137 |
| | | | | BIP(B1c+B2) | TGTTGTAGCTTGTCACACCGTTCCACACATGACCATTTCACT | | 42 | 138 |
| | | | 1 890 | LoopF | TAAGCATGTTAGGCATGGCT | 1 890 | 20 | 139 |
| | | | 1 964 | LoopB | AGCTAATGAGTGTGCTCAAGT | 1 964 | 21 | 140 |
| | | | 1 844 | F2 | GGGTTGGGATTATCCTAAATGT | 1 844 | 22 | 141 |
| | | | 1 920 | F1c | TGCGAGCAAGAACAAGIGAGG | 1 920 | 21 | 142 |
| | | | 2 008 | B2 | CCACACATGACCATTTCACT | 2 008 | 20 | 143 |
| | | | 1932 | B1c | TGTTGTAGCTTGTCACACCGTT | 1932 | 22 | 144 |
| | | | | Product | GGGTTGGGATTATCCTAAATGTGATAGAGCCATGCCTAACATGCTTAG AATTATGGCCTCACTTGTTCTTGCTCGCAAACATACAACGTGTTGTAGC TTGTCACACCGTTTCTATAGATTAGCTAATGAGTGTGCTCAAGTATTGA GTGAAATGGTCATGTGTGG | | 165 | 145 |
| **N** | SARS-CoV-2 | Rd Rp gene | 1 899 | F3 | GCCTCACTTGTTCTTGCT | 1 899 | 18 | 146 |
| | (NC_045512) | | 2 161 | B3 | CGGACATACTTATCGGCAAT | 2 161 | 20 | 147 |
| | | | | FIP(F1c+F2) | AACCGCCACACATGACCATTTTGTAGCTTGTCACACCG | | 38 | 148 |
| | | | | BIP(B1c+B2) | AGGTGGAACCTCATCAGGAGACCGTGACAGCTTGACAAA | | 39 | 149 |
| | | | 1 993 | LoopF | TCACTCAATACTTGAGCACACT | 1 993 | 22 | 150 |
| | | | 2 051 | LoopB | TGCCACAACTGCTTATGCTA | 2 051 | 20 | 151 |
| | | | 1934 | F2 | TTGTAGCTTGTCACACCG | 1 934 | 18 | 152 |
| | | | 2 013 | F1c | AACCGCCACACATGACCATT | 2 013 | 20 | 153 |
| | | | 2 103 | B2 | CCGTGACAGCTTGACAAA | 2 103 | 18 | 154 |
| | | | 2 030 | B1c | AGGTGGAACCTCATCAGGAGA | 2 030 | 21 | 155 |
| | | | | Product | TTGTAGCTTGTCACACCGTTTCTATAGATTAGCTAATGAGTGTGCTCAA GTATTGAGTGAAATGGTCATGTGTGGCGGTTCACTATATGTTAAACCA GGTGGAACCTCATCAGGAGATGCCACAACTGCTTATGCTAATAGTGTT TTTAACATTTGTCAAGCTGTCACGG | | 170 | 156 |
| **O** | SARS-CoV-2 | Rd Rp gene | 1 899 | F3 | GCCTCACTTGTTCTTGCT | 1 899 | 18 | 157 |
| | (NC_045512) | | 2 161 | B3 | CGGACATACTTATCGGCAAT | 2 161 | 20 | 158 |
| | | | | FIP(F1c+F2) | CCGCCACACATGACCATTTCTGTTGTAGCTTGTCACACC | | 39 | 159 |
| | | | | BIP(B1c+B2) | AGGTGGAACCTCATCAGGAGACCGTGACAGCTTGACAAA | | 39 | 160 |
| | | | 1 991 | LoopF | ACTCAATADTTCAGCACACTCA | 1 991 | 22 | 161 |
| | | | 2 051 | LoopB | TGCCACAACTGCTTATGCTA | 2 051 | 20 | 162 |
| | | | 1 932 | F2 | TGTTGTAGCTTGTCACACC | 1 932 | 19 | 163 |
| | | | 2 011 | F1c | CCGCCACACATGACCATTTC | 2 011 | 20 | 164 |
| | | | 2103 | B2 | CCGTGACAGCTTGACAAA | 2103 | 18 | 165 |
| | | | 2 030 | B1c | AGGTGGAACCTCATCAGGAGA | 2 030 | 21 | 166 |
| | | | | Product | TGTTGTAGCTTGTCACACCGTTTCTATAGATTAGCTAATGAGTGTGCTC AAGTATTGAGTGAAATGGTCATGTGTGGCGGTTCACTATATGTTAAAC CAGGTGGAACCTCATCAGGAGATGCCACAACTGCTTATGCTAATAGTG TTTTTAACATTTGTCAAGCTGTCACGG | | 172 | 167 |
| **P** | SARS-CoV-2 | Rd Rp gene | 1 838 | F3 | CCTTATGGGTTGGGATTATCC | 1 838 | 21 | 168 |
| | (NC_045512) | | 2 114 | B3 | ATTAACATTGGCCGTGACA | 2 114 | 19 | 169 |
| | | | | FIP(F1c+F2) | AACGGTGTGACAAGCTACAACAGCTTAGAATTATGGCCTCACT | | 43 | 170 |
| | | | | BIP(B1c+B2) | GTCATGTGTGGCGGTTCACTAAAGCAGTTGTGGCATCTC | | 39 | 171 |
| | | | 1 927 | LoopF | GTATGTTTGCGAGCAAGAACA | 1 927 | 21 | 172 |
| | | | 2 028 | LoopB | CCAGGTGGAACCTCATCAG | 2 028 | 19 | 173 |
| | | | 1 886 | F2 | GCTTAGAATTATGGCCTCACT | 1 886 | 21 | 174 |
| | | | 1 953 | F1c | AACGGTGTGACAAGCTACAACA | 1 953 | 22 | 175 |
| | | | 2 064 | B2 | AAGCAGTTGTGGCATCTC | 2 064 | 18 | 176 |
| | | | 1 998 | B1c | GTCATGTGTGGCGGTTCACTA | 1998 | 21 | 177 |
| | | | | Product | GCTTAGAATTATGGCCTCACTTGTTCTTGCTCGCAAACATACAACGTGT TGTAGCTTGTCACACCGTTTCTATAGATTAGCTAATGAGTGTGCTCAAG TATTGAGTGAAATGGTCATGTGTGGCGGTTCACTATATGTTAAACCAG GTGGAACCTCATCAGGAGATGCCACAACTGCTT | | 179 | 178 |
| **T** | SARS-CoV-2 | RdRp gene | 1 838 | F3 | CCTTATGGGTTGGGATTATCC | 1838 | 21 | 179 |
| | (NC_045512) | | 2 057 | B3 | TGTGGCATCTCCTGATGA | 2057 | 18 | 180 |
| | | | | FIP(F1c+F2) | CACGTTGTATGTTTGCGAGCAAGTGATAGAGCCATGCCTAAC | | 42 | 181 |
| | | | | BIP(B1c+B2) | TGTAGCTTGTCACACCGTTTCTCCACACATGACCATTTCACT | | 42 | 182 |
| | | | 1 906 | LoopF | AGTGAGGCCATAATTCTAAGCA | 1906 | 22 | 183 |
| | | | 1964 | LoopB | AGCTAATGAGTGTGCTCAAGT | 1964 | 21 | 184 |
| | | | 1864 | F2 | GTGATAGAGCCATGCCTAAC | 1864 | 20 | 185 |
| | | | 1933 | F1c | CACGTTGTATGTTTGCGAGCAA | 1933 | 22 | 186 |
| | | | 2008 | B2 | CCACACATGACCATTTCACT | 2008 | 20 | 187 |
| | | | 1935 | B1c | TGTAGCTTGTCACACCGTTTCT | 1935 | 22 | 188 |
| | | | | Product | GTGATAGAGCCATGCCTAACATGCTTAGAATTATGGCCTCACTTGTTCT TGCTCGCAAACATACAACGTGTTGTAGCTTGTCACACCGTTTCTATAGA TTAGCTAATGAGTGTGCTCAAGTATTGAGTGAAATGGTCATGTGTGG | | 145 | 189 |
| **U** | SARS-CoV-2 | RdRp gene | 1838 | F3 | CCTTATGGGTTGGGATTATCC | 1 838 | 21 | 190 |
| | (NC_045512) | | 2057 | B3 | TGTGGCATCTCCTGATGA | 2 057 | 18 | 191 |
| | | | | FIP(F1c+F2) | CACGTTGTATGTTTGCGAGCAAAATGTGATAGAGCCATGCC | | 41 | 192 |
| | | | | BIP(B1c+B2) | TGTAGCTTGTCACACCGTTTCTCCACACATGACCATTTCACT | | 42 | 193 |
| | | | 1906 | LoopF | AGTGAGGCCATAATTCTAAGCA | 1 906 | 22 | 194 |
| | | | 1964 | LoopB | AGCTAATGAGTGTGCTCAAGT | 1 964 | 21 | 195 |
| | | | 1861 | F2 | AATGTGATAGAGCCATGCC | 1 861 | 19 | 196 |
| | | | 1933 | F1c | CACGTTGTATGTTTGCGAGCAA | 1 933 | 22 | 197 |
| | | | 2008 | B2 | CCACACATGACCATTTCACT | 2 008 | 20 | 198 |
| | | | 1935 | B1c | TGTAGCTTGTCACACCGTTTCT | 1935 | 22 | 199 |
| | | | | Product | AATGTGATAGAGCCATGCCTAACATGCTTAGAATTATGGCCTCACTTG TTCTTGCTCGCAAACATACAACGTGTTGTAGCTTGTCACACCGTTTCTA TAGATTAGCTAATGAGTGTGCTCAAGTATTGAGTGAAATGGTCATGTG TGG | | 148 | 200 |
| **V** | SARS-CoV-2 | RdRp gene | 1 838 | F3 | CCTTATGGGTTGGGATTATCC | 1838 | 21 | 201 |
| | (NC_045512) | | 2 057 | B3 | TGTGGCATCTCCTGATGA | 2057 | 18 | 202 |
| | | | | FIP(F1c+F2) | CACGTTGTATGTTTGCGAGCAAATGTGATAGAGCCATGCCTA | | 42 | 203 |
| | | | | BIP(B1c+B2) | TGTAGCTTGTCACACCGTTTCTCCACACATGACCATTTCACT | | 42 | 204 |
| | | | 1906 | LoopF | AGTGAGGCCATAATTCTAAGCA | 1906 | 22 | 205 |
| | | | 1964 | LoopB | AGCTAATGAGTGTGCTCAAGT | 1964 | 21 | 206 |
| | | | 1 862 | F2 | ATGTGATAGAGCCATGCCTA | 1862 | 20 | 207 |
| | | | 1933 | F1c | CACGTTGTATGTTTGCGAGCAA | 1933 | 22 | 208 |
| | | | 2 008 | B2 | CCACACATGACCATTTCACT | 2008 | 20 | 209 |
| | | | 1935 | B1c | TGTAGCTTGTCACACCGTTTCT | 1935 | 22 | 210 |
| | | | | Product | ATGTGATAGAGCCATGCCTAACATGCTTAGAATTATGGCCTCACTTGTT CTTGCTCGCAAACATACAACGTGTTGTAGCTTGTCACACCGTTTCTATA GATTAGCTAATGAGTGTGCTCAAGTATTGAGTGAAATGGTCATGTGTG G | | 147 | 211 |
| **AC** | SARS-CoV-2 | ORF3a + E 610 | | F3 | CACAGTTACTTCACTTCAGACT | 610 | 22 | 212 |
| | (NC_045512) | | 960 | B3 | CGCAGTAAGGATGGCTAG | 960 | 18 | 213 |
| | | | | FIP(F1c+F2) | GCTAGTAGTCGTCGTCGGTTCTGATGAGCCTGAAGAACATG | | 41 | 214 |
| | | | | BIP(B1c+B2) | AAGCACAAGCTGATGAGTACGAGTAACTAGCAAGAATACCACGA | | 44 | 215 |
| | | | 759 | LoopF | GGATGAACCGTCGATTGTGT | 759 | 20 | 216 |
| | | | 867 | LoopB | TTCGGAAGAGACAGGTACG | 867 | 19 | 217 |
| | | | 711 | F2 | TGATGAGCCTGAAGAACATG | 711 | 20 | 218 |
| | | | 816 | F1c | GCTAGTAGTCGTCGTCGGTTC | 816 | 21 | 219 |
| | | | 941 | B2 | GTAACTAGCAAGAATACCACGA | 941 | 22 | 220 |
| | | | 827 | B1c | AAGCACAAGCTGATGAGTACGA | 827 | 22 | 221 |

| **Table 1. RT-LAMP assays p rimer list** | | | | | | | | |
|---|---|---|---|---|---|---|---|---|
| **Primer Set ID** | **Target genome (accession nr)** | **Target region** | **Position** | **Name** | **Sequence (5' to 3')** | **Primer position** | **Primer length** | **SEQ ID NO:** |
| | | | | Product | TGATGAGCCTGAAGAACATGTCCAAATTCACACAATCGACGGTTCATC CGGAGTTGTTAATCCAGTAATGGAACCAATTTATGATGAACCGACGAC GACTACTAGCGTGCCTTTGTAAGCACAAGCTGATGAGTACGAACTTAT GTACTCATTCGTTTCGGAAGAGACAGGTACGTTAATAGTTAATAGCGT ACTTCTTTTTCTTGCTTTCGTGGTATTCTTGCTAGTTAC | | 231 | 222 |

## Claims

1. A first set of oligonucleotide primers for amplification of Severe acute respiratory syndrome coronavirus 2 (SARS-CoV-2) RNA which comprises at least a forward primer and a reverse primer targeting SARS-CoV-2 Nucleocapsid (N) gene selected from the group consisting of:
- a forward primer which hybridizes to a sequence of at least 10 consecutive nucleotides, preferably at least 15 consecutive nucleotides from positions 486 to 505, 73 to 91, 171 to 189, 208 to 227, 450 to 467, 468 to 487, or 538 to 555 of said N gene complement sequence; and
- a reverse primer which hybridizes to a sequence of at least 10 consecutive nucleotides, preferably at least 15 consecutive nucleotides from positions 752 to 771, 307 to 328, 450 to 467, 450 to 467, 675 to 674, 739 to 759, or 762 to 779, respectively of said N gene sequence; and the indicated positions being determined by alignment with SEQ ID NO: 1.

2. The first set of primers according to claim 1, which is for isothermal nucleic acid amplification of SARS-CoV-2 RNA and which contains primers comprising sequences selected from the group consisting of: the sequences SEQ ID NO: 47 to 52 or variants thereof; the sequences SEQ ID NO: 3 to 8 or variants thereof; the sequences SEQ ID NO: 14 to 19 or variants thereof; the sequences SEQ ID NO: 25 to 30 or variants thereof; the sequences SEQ ID NO: 36 to 41 or variants thereof; the sequences SEQ ID NO: 58 to 63 or variants thereof; the sequences SEQ ID NO: 69 to 74 or variants thereof; the sequences SEQ ID NO: 80 to 85 or variants thereof; the sequences SEQ ID NO: 91 to 96 or variants thereof; the sequences SEQ ID NO: 102 to 107 or variants thereof; and the sequences SEQ ID NO: 113 to 118 or variants thereof.

3. The first set of primers according to claim 1 or 2, which is specific for SARS-CoV-2.

4. A second set of oligonucleotide primers for amplification of Severe acute respiratory syndrome coronavirus 2 (SARS-CoV-2) RNA which amplifies a target sequence of at least 100 nucleotides of SARS-CoV-2 RNA-dependent RNA polymerase (RdRp) gene situated between any one of positions 1819 to 1838 and any one of positions 2057 to 2076; any one of positions 1763 to 1782 and any one of positions 2057 to 2076; any one of positions 1819 to 1838 and any one of positions 2114 to 2133; and any one of positions 1880 to 1899 and any one of positions 2161 to 2180 of said RdRp gene sequence; and the indicated positions being determined by alignment with SEQ ID NO: 2.

5. The second set of oligonucleotide primers according to claim 4, which comprises at least a forward and a reverse primer targeting SARS-CoV-2 RNA-dependent RNA polymerase (RdRp) gene selected from the group consisting of:
- a forward primer which hybridizes to a sequence of at least 10 consecutive nucleotides, preferably at least 15 consecutive nucleotides from positions 1838 to 1858, 1782 to 1799, 1838 to 1858, or 1899 to 1916 of said RdRp gene complement sequence; and
- a reverse primer which hybridizes to a sequence of at least 10 consecutive nucleotides, preferably at least 15 consecutive nucleotides from positions 2040 to 2057, 2040 to 2057, 2096 to 2114, or 2142 to 2161, respectively of said RdRp gene sequence; and the indicated positions being determined by alignment with SEQ ID NO: 2.

6. The second set of primers according to claim 5, which is for isothermal nucleic acid amplification of SARS-CoV-2 RNA and which contains primers comprising sequences selected from the group consisting of: the sequences SEQ ID NO: 179 to 184 or variants thereof; the sequences SEQ ID NO: 124 to 129 or variants thereof; the sequences SEQ ID NO: 135 to 140 or variants thereof; the sequences SEQ ID NO: 146 to 151 or variants thereof; the sequences SEQ ID NO: 157 to 162 or variants thereof; the sequences SEQ ID NO: 168 to 173 or variants thereof; the sequences SEQ ID NO: 190 to 195 or variants thereof; and the sequences SEQ ID NO: 201 to 206 or variants thereof.

7. The second set of primers according to any one of claims 4 to 6, which is specific for Betacoronavirus group B/C.

8. The first or second set of oligonucleotide primers according to any one of claims 1 to 7, which comprise at least one labelled oligonucleotide primer; preferably a fluorescent oligonucleotide primer.

9. A combination of oligonucleotide primers comprising at least a first set of primers specific for SARS-CoV-2 according to claim 3 or 8 and a second set of primers specific for Betacoronavirus group B/C.according to claim 7 or 8.

10. The combination of claim 9, which contains a first set primers comprising sequences selected from the group consisting of the sequences SEQ ID NO: 47 to 52 or variants thereof and a second set of primers comprising sequences selected from the group consisting of the sequences SEQ ID NO: 179 to 184 or variants thereof.

11. The combination of claim 9 or 10, which provides a differential diagnosis between infection and disease caused by SARS-CoV-2 and other Betacoronavirus group B/C such as SARS-CoV and MERS-CoV.

12. A kit for the detection of SARS-CoV-2 and/or Betacoronavirus group B/C RNA, comprising at least one set of oligonucleotide primers or a combination thereof according to any one of claims 1 to 11.

13. Use of the kit according to claim 12, for the *in vitro* diagnosis of SARS-CoV-2 and/or Betacoronavirus group B/C infection and associated disease in a biological sample from a subject.

14. Use of the kit according to claim 12, for the *in vitro* detection of a contamination with SARS-CoV-2 and/or Betacoronavirus group B/C in an environmental sample.

15. A method of detection of SARS-CoV-2 and/or Betacoronavirus group B/C RNA in a sample, comprising :
- subjecting said sample to an isothermal nucleic acid amplification reaction using a set of primers according to any one of claims 2-3 and 6 to 8 for amplifying a target sequence of said viral RNA, and
- detecting the presence of an amplification product for said target sequence.
